# EUROPÄISCHE PATENTSCHRIFT

(11) **EP 1 758 914 B1**
(45) Veröffentlichungstag und Bekanntmachung des Hinweises auf die Patenterteilung: **21.11.2007**
(21) Anmeldenummer: 05746637.7
(22) Anmeldetag: 03.06.2005
(51) Int. Cl.: C07H 17/02, A61K 31/7056, A61P 3/10

(54) **NEUE FLUORGLYKOSIDDERIVATE VON PYRAZOLEN, DIESE VERBINDUNGEN ENTHALTENDE ARZNEIMITTEL UND DEREN VERWENDUNG**
NOVEL FLUOROGLYCOSIDE DERIVATIVES OF PYRAZOLES, MEDICAMENTS CONTAINING THESE COMPOUNDS, AND THE USE THEREOF
NOUVEAUX DERIVES FLUOROGLYCOSIDIQUES DE PYRAZOLES, MEDICAMENTS CONTENANT CES COMPOSES ET LEUR UTILISATION

(30) Priorität: 11.06.2004 DE 102004028241
(43) Veröffentlichungstag der Anmeldung: 07.03.2007
(73) Patentinhaber: Sanofi-Aventis Deutschland GmbH, 65929 Frankfurt am Main (DE)
(72) Erfinder: BRUMMERHOP, Harm, 60389 Frankfurt (DE); FRICK, Wendelin, 65510 Hünstetten-Beuerbach (DE); GLOMBIK, Heiner, 65719 Hofheim (DE); PLETTENBURG, Oliver, 65799 Kelkheim (DE); BICKEL, Martin, 61348 Bad Homburg (DE); HEUER, Hubert, 55270 Schwabenheim (DE); THEIS, Stefan, 60594 Frankfurt (DE)
(86) Internationale Anmeldenummer: PCT/EP2005/005959
(87) Internationale Veröffentlichungsnummer: WO 2005/121161

(56) Entgegenhaltungen:
- EP-A- 0 850 948
- EP-A- 1 213 296
- WO-A-20/04052903

## Beschreibung

Die Erfindung betrifft substituierte Fluorglykosidderivate von Pyrazolen, deren physiologisch verträgliche Salze sowie physiologisch funktionelle Derivate.

In der Literatur sind bereits mehrere Substanzklassen mit SGLT-Wirkung bekannt. All diesen Strukturen diente als Leitbild der Naturstoff Phlorizin. Von diesem wurden folgende Klassen abgeleitet, die in den nachfolgenden Schutzrechten beschrieben sind:
- Propiophenonglykoside von Tanabe (WO 0280936, WO 0280935, JP 2000080041 und EP 850948)
- 2-(Glucopyranoslyoxy)-benzylbenzole von Kissei (WO 0244192, WO 0228872, WO 03011880 und WO 0168660)
- Glucopyranosyloxy-pyrazole von Kissei, Bristol-Myers Squibb und Ajinomoto (WO 02068440, WO 02068439, WO 0236602, WO 01016147, WO 02053573, WO 03020737, WO 03090783, WO 04014932, WO 04019958 und WO 04018491)
- O-Glykosidbenzamide von Bristol-Myers Squibb (WO 0174835 und WO 0174834).
- Glucopyranosyloxy-thiophene von Aventis (WO 04007517)
- und C-Arylglykoside von Bristol-Myers Squibb (WO 03099836, WO 0127128 und US 2002137903).

Alle bekannten Strukturen enthalten als sehr wichtiges Strukturelement die Glucose.

Der Erfindung lag die Aufgabe zugrunde, neue Verbindungen zur Verfügung zu stellen, mit denen eine Prävention und Behandlung von Diabetes Typ 1 und Typ 2 möglich ist. Wir haben nun überraschenderweise gefunden, dass Fluorglykosidderivate von Pyrazolen die Wirkung auf SGLT steigern. Diese Verbindungen eignen sich daher besonders zur Prävention und Behandlung von Diabetes Typ 1 und Typ 2.

Die Erfindung betrifft daher Verbindungen der Formel I, 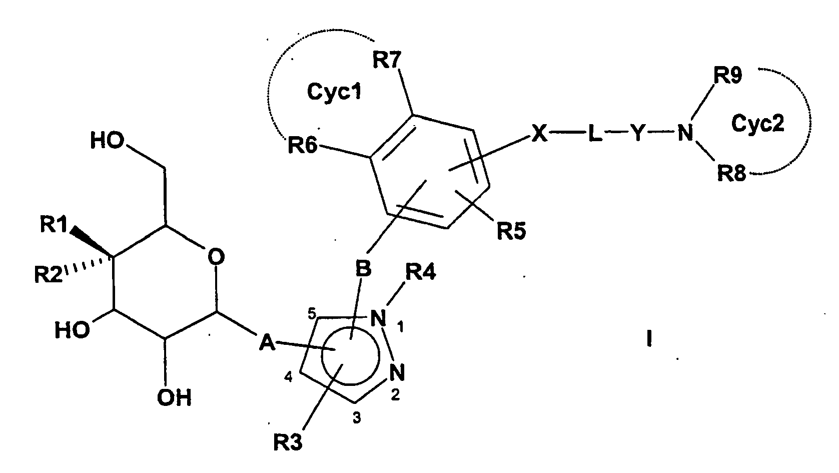 worin bedeuten
- R1 und R2: unabhängig voneinander, F oder H, wobei einer der Reste R1 oder R2 F sein muss;
- A: O, NH, CH₂, S oder eine Bindung;
- R3: Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁-C₆)-Alkyl, (C₃-C₆)-Cycloalkyl, (C₂-C₆)-Alkenyl, (C₂-C₆)-Alkinyl, O-(C₁-C₆)-Alkyl, HO-(C₁-C₆)Alkylen, (C₁-C₆)-Alkylen-O-(C₁-C₆)-Alkyl, Phenyl, Benzyl, (C₁-C₆)-Alkoxycarboxyl, wobei in den Alkyl-, Alkenyl-, Alkinyl- bzw. O-Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH-CO-(C₁-C₇)-Alkyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl ; NH₂, NH(C₁-C₆)-Alkyl, N((C₁-C₆)-Alkyl)₂, SO₂-CH₃, COOH, COO-(C₁-C₆)-Alkyl, CONH₂;
- R4: Wasserstoff, (C₁-C₆)-Alkyl, (C₂-C₆)-Alkenyl, (C₃-C₆)-Cycloalkyl, oder Phenyl, dass gegebenenfalls durch Halogen oder (C₁-C₄)-Alkyl substituiert sein kann;
- B: (C₀-C₁₅)-Alkylen, wobei ein oder mehrere C-Atome des Alkylen-Rests unabhängig voneinander durch -O-, -(C=O)-, -CH=CH-, -C≡C-, -S-, -CH(OH)-, -CHF-, -CF₂-, -(S=O)-, -(SO₂)- -N((C₁-C₆)-Alkyl)-, -N((C₁-C₆)-Alkyl-Phenyl)- oder -NH- ersetzt sein können;
- R5, R6, R7: unabhängig voneinander Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁-C₆)-Alkyl, (C₂-C₆)-Alkenyl, (C₂-C₆)-Alkinyl, O-(C₁-C₈)-Alkyl, HO-(C₁-C₆)-Alkylen, (C₁-C₆)-Alkylen-O-(C₁-C₆)-Alkyl, wobei in den Alkyl-, Alkenyl, Alkinyl bzw. O-Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₒ-Phenyl, SCF₃, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH-CO-(C₁-C₆)-Alkyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₈)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
oder
- R6 und R7: gemeinsam mit den sie tragenden C-Atomen ein 5 bis 7 gliedrigen, gesättigter, teilweise oder vollständig ungesättigter Ring Cyc1, wobei 1 oder 2 C-Atom(e) des Ringes auch durch N, O oder S ersetzt sein können und Cyc1 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁-C₄)-Alkyl, OCF₃ substituiert sein kann;
- X: CO, O, NH, S, SO, SO₂ oder eine Bindung;
- L: (C₁-C₆)-Alkylen, (C₂-C₅)-Alkenylen, (C₂-C₅)-Alkinylen, wobei jeweils eine oder zwei CH₂-Gruppe(n) durch O oder NH ersetzt sein kann;
- Y: CO, NHCO, SO, SO₂, oder eine Bindung;
- R8, R9: unabhängig voneinander Wasserstoff, SO₃H, Zuckerrest, (C₁-C₆)-Alkyl, wobei ein oder mehrere CH₂-Gruppen des Alkyl-Rests unabhängig voneinander durch (C₁-C₆)-Alkyl, OH, (C₁-C₆)-Alkylen-OH, (C₂-C₆)-Alkenylen-OH, O-Zuckerrest, OSO₃H, NH₂, NH-(C₁-C₆)-Alkyl], N[(C₁-C₆)-Alkyl]₂, NH-CO-(C₁-C₆)-Alkyl, NH-Zuckerrest, NH-SO₃H, (C₁-C₆)-Alkylen-NH₂, (C₂-C₆)-Alkenylen-NH₂, (C₀-C₆)-Alkylen-COOH, (C₀-C₆)-Alkylen-CONH₂, (C₀-C₆)-Alkylen-CONH-(C₁-C₆)-Alkyl, (C₀-C₆)-Alkylen-SONH₂, (C₀-C₆)-Alkylen-SONH-(C₁-C₆)-Alkyl, (C₀-C₆)-Alkylen-SO₂NH₂, (C₀-C₆)-Alkylen-SO₂NH-(C₁-C₆)-Alkyl, Adamantyl substituiert sein können; oder
- R8 und R9: gemeinsam mit den sie tragenden N-Atom ein 5 bis 7 gliedrigen, gesättigten Ring Cyc2 bilden, wobei ein oder mehrere CH₂-Gruppen des Ringes auch durch O, S, NH, NSO₃H, N-Zuckerrest,
N-(C₁-C₆)-Alkyl, ersetzt sein kann, wobei ein oder mehrere CH₂-Gruppen des Alkyl-Rests unabhängig voneinander durch (C₁-C₆)-Alkyl, OH, (C₁-C₆)-Alkylen-OH, (C₂-C₆)-Alkenylen-OH, NH₂, NH-(C₁-C₆)-Alkyl, N[(C₁-C₆)-Alkyl]₂, NH-CO-(C₁-C₆)-Alkyl, NH-Zuckerrest, (C₁-C₆)-Alkylen-NH₂, (C₂-C₆)-Alkenylen-NH₂, (C₀-C₆)-Alkylen-COOH, (C₀-C₆)-Alkylen-CONH₂, (C₀-C₆)-Alkylen-CONH-(C₁-C₆)-Alkyl, (C₀-C₆)-Alkylen-SONH₂, (C₀-C₆)-Alkylen-SONH-(C₁-C₆,)-Alkyl, (C₀-C₆)-Alkylen-SO₂NH₂, (C₀-C₆)-Alkylen-SO₂NH-(C₁-C₆)-Alkyl substituiert sein können,
sowie deren pharmazeutisch verträgliche Salze.

Unter Zuckerresten werden Verbindungen verstanden, die sich von Aldosen und Ketosen mit 3 bis 7 Kohlenstoffatomen ableiten, die der D- oder L-Reihe angehören können; dazu gehören auch Aminozucker, Zuckeralkohole oder Zuckersäuren (Jochen Lehmann, Chemie der Kohlenhydrate, Thieme Verlag 1976). Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure, Mannonsäure, Glucamin, 3-Amino-1,2-propandiol, Glucarsäure und Galaktarsäure. Die Bindungen können dabei in der alpha- und beta-Form auftreten.

Die Verknüpfungspunkte von A, B, R3 und R5 an den Ring sind frei wählbar. Alle resultierenden Verbindungen der Formel I gehören zur vorliegenden Erfindung.

Bevorzugt sind Verbindungen der Formel I, worin bedeuten
- A: O, NH, eine Bindung;
- R3: Wasserstoff, F, Cl, Br, J, OH, CF₃, NO₂, CN, COOH, CO-(C₁-C₆)-Alkyl, COO-(C₁-C₆)-Alkyl, CONH₂, CONH-(C₁-C₆)-Alkyl, CO-N[(C₁-C₆),-Alkyl]₂, (C₁-C₆)-Alkyl, (C₂-C₆)-Alkenyl, (C₂-C₆)-Alkinyl, O-(C₁-C₆)-Alkyl, HO-(C₁-C₆)-Alkylen, (C₁-C₆)-Alkylen-O-(C₁-C₆)-alkyl, Phenyl, Benzyl, (C₁-C₄)-Alkylen-COOH, SO-(C₁-C₆)-Alkyl, wobei in den Alkylresten ein, mehrere, oder alle Wasserstoff(e) durch Fluor ersetzt sein können; oder
- R4: Wasserstoff, (C₁-C₆)-Alkyl, (C₁-C₆)-Alkenyl, (C₃-C₆)-Cycloalkyl;
- B: (C₀-C₆)-Alkylen, wobei ein oder mehrere C-Atom(e) des Alkylen-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkylen)-, -N((C₁-C₆)-Alkylen-Phenylen)- oder-NH- ersetzt sein können.

Bevorzugt sind ferner Verbindungen der Formel I, in denen die Zucker-Reste beta(β)-verknüpft sind und die Stereochemie in 2-, 3- und 5-Position des Zuckerrestes D-gluco-konfiguriert ist.

Bevorzugt sind ferner Verbindungen der Formel I, in denen
- R1: Wasserstoff und
- R2: Fluor,
oder
- R1: Fluor und
- R2: Wasserstoff;
- A: O, NH;
- R3: Wasserstoff, F, Cl, Br, J, OH, CF₃, (C₁-C₆)-Alkyl, (C₃-C₆)-Cycloalkyl, (C₂-C₆)-Alkenyl, O-(C₁-C₆)-Alkyl, wobei in den Alkylresten ein, mehrere, oder alle Wasserstoff(e) durch Fluor ersetzt sein können;
- R4: Wasserstoff, (C₁-C₆)-Alkyl, (C₃-C₆)-Cycloalkyl;
- B: (C₀-C₄)-Alkylen, wobei ein oder mehrere C-Atom(e) des Alkylen-Rests unabhängig voneinander durch -O-, -(C=O)-, -CH=CH-, - CH(OH)-, CHF-, -CF₂- oder -NH- ersetzt sein können;
- R5, R6, R7: unabhängig voneinander Wasserstoff, F, Cl, Br, J, OH, CF₃, NO₂, CN, COOH, COO(C₁-C₆)-Alkyl, CO(C₁-C₄)-Alkyl, CONH₂, CONH-(C₁-C₆)-Alkyl, CO-N[(C₁-C₆)-Alkyl]₂, (C₁-C₆)-Alkyl, (C₂-C₆)-Alkenyl, (C₂-C₆-Alkinyl, O-(C₁-C₈)-Alky), HO-(C₁-C₆)-Alkylen, (C₁-C₆)-Alkylen-O-(C₁-C₆)-Alkyl, wobei in den Alkyl-, Alkenyl-, Alkinyl- bzw. O-Alkylresten ein, mehrere, oder alle Wasserstoff(e) durch Fluor ersetzt sein können;
NH₂, NH-(C₁-C₆)-Alkyl, N((C₁-C₆)-Alkyl)₂, NH-CO-(C₁-C₆)-Alkyl, oder
- R6 und R7: gemeinsam mit den sie tragenden C-Atomen ein 5 bis 7 gliedrigen, gesättigter, teilweise oder vollständig ungesättigter Ring Cyc1, wobei 1 oder 2 C-Atom(e) des Ringes auch durch N, O oder S ersetzt sein können und Cyc1 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁-C₄)-Alkyl, OCF₃ substituiert sein kann;
- X: CO, O, NH, eine Bindung;
- L: (C₁-C₆)-Alkylen, (C₂-C₅)-Alkenylen, wobei jeweils eine oder zwei CH₂-Gruppe(n) durch O oder NH ersetzt sein kann;
- Y: CO, NHCO, eine Bindung bedeuten.

Besonders bevorzugt sind Verbindungen der Formel I, in denen
- R1: Wasserstoff;
- R2: Fluor;
- A: O;
- R3: CF₃, Methyl, Isopropyl;
- R4: Wasserstoff;
- B: (C₀-C₄)-Alkylen, wobei ein oder mehrere C-Atom(e) des Alkylen-Rests unabhängig voneinander durch -O-, -(C=O)-, -CHF- oder -CF₂- ersetzt sein können;
- X: CO, O, eine Bindung;
- L: (C₁-C₄)-Alkylen, (C₂-C₄)-Alkenylen, wobei jeweils eine oder zwei CH₂-Gruppe(n) durch O oder NH ersetzt sein kann;
- Y: CO, NHCO, eine Bindung
bedeuten.

Ganz besonders bevorzugt sind Verbindungen der Formel 1, in denen
- R1: Wasserstoff;
- R2: Fluor;
- A: O;
- B: -CH₂-;
- R5: Wasserstoff, CI, Methyl, Ethyl, OH, CF₃;
- R6, R7: Wasserstoff;
- X: CO, O eine Bindung;
- L: (C₁-C₃)-Alkylen, (C₂-C₃)-Alkenylen, wobei jeweils eine CH₂-Gruppe durch O oder NH ersetzt sein kann;
- Y: CO, NHCO, eine Bindung bedeuten.

Besonders bevorzugt sind Verbindungen der Formel I, in denen die Substituenten A und B eine benachbarte Stellung (ortho-Stellung) einehmen und R3 eine benachbarte Stellung (ortho-Stellung) zu B einnimmt.

Weiterhin sind ganz besonders bevorzugt Verbindungen der Formel I, in denen
- R8, R9: unabhängig voneinander Wasserstoff, SO₃H, Zuckerrest, (C₁-C₄)-Alkyl, wobei der Alkyl-Rest unabhängig voneinander ein oder mehrfach durch (C₁-C₂)-Alkyl, OH, (C₁-C₂)-Alkylen-OH, OSO₃H, NH₂, CONH₂, SO₂NH₂, NH-SO₃H oder Adamantyl substituiert sein kann;
oder
- R8 und R9: gemeinsam mit den sie tragenden N-Atom ein 5 bis 7 gliedrigen, gesättigten Ring Cyc2 bilden, ausgewählt aus der Gruppe Piperazin, welches N-subtituiert sein kann durch (C₁-C₂)-Alkyl, (C₁-C₂)-Alkylen-OH oder SO₃H,
Piperidin, Azepan, Pyrrolidin oder Morpholin bedeuten.

In einer besonderen Ausführungsform der Verbindungen der Formel I sind die Substituenten B und X in para-Position am Phenylring angeordnet.

In einer weiteren Ausführungsform der Verbindungen der Formel I sind die Substituenten A in 3-Position, B in 4-Position und R3 in 5-Position am Pyrazolring angeordnet.

In einer weiteren Ausführungsform der Verbindungen der Formel I sind die Substituenten A in 5-Position, B in 4-Position und R3 in 3-Position am Pyrazolring angeordnet.

Die Alkylreste in den Substituenten R3, R4, R5, R6, R7, R8 und R9 können sowohl geradkettig wie verzweigt sein. Unter Halogen werden F, Cl, Br, J, bevorzugt F und Cl verstanden.

Die Erfindung bezieht sich auf Verbindungen der Formel I, in Form ihrer Tautomere, Racemate, racemischen Mischungen und reinen Enantiomere sowie auf ihre Diastereomere und Mischungen davon. Die vorliegende Erfindung umfasst alle diese isomeren und ggf. tautomeren Formen der Verbindungen der Formel 1. Diese isomeren Formen können, wenn auch (zum Teil) nicht expressis verbis beschrieben, nach bekannten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bemstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium-und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

### Verwendung.

Diese Erfindung bezieht sich weiterhin auf die Verwendung von Verbindungen der Formel I und ihren pharmazeutischen Zusammensetzungen zur Inhibierung des SGLT 1 (sodium dependent glucose transporter 1). Bei der intestinalen Aufnahme von Kohlenhydraten, insbesondere der intestinalen Aufnahme von Glukose, ist SGLT1 beteiligt (E. Turk et al., Nature 1991, 350, 354-356.). Eine Inhibierung der Absorption von Glukose inhibiert den Anstieg der Blutglukose-Konzentration. Somit sind Inhibitoren von SGLT1 zur Behandlung, Kontrolle und Prophylaxe von metabolischen Erkrankungen, inbesondere von Diabetes mellitus, geeignet.

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.

Die Verbindungen der Formel I eignen sich weiterhin zur Prävention und Behandlung von Diabetischen Spätschäden, wie z.B. Nephropatie, Retinopathie, Neuropathie sowie Syndrom X, Obesitas, Herzinfarkt, Myocardialem Infarkt, peripheren arteriellen Verschlusskrankheiten, Thrombosen, Arteriosklerose, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie zur Prävention und Behandlung von Diabetischen Spätschäden, Syndrom X und Obesitas.

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, perorale (z.B. sublinguale) und Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel 1 enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel 1 insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

### Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221;633), GLP-1-Rezeptor Modulatoren, wie in WO 01/04146 beschrieben, oder auch wie z.B. diejenigen, die in WO 98/08871 von Novo Nordisk A/S offenbart wurden.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, orale GLP-1-Agonisten, DPP-IV Inhibitoren, Kaliumkanalöffner, wie z.B. diejenigen, die in WO 97/26265 und WO 99/03861 offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen, die zur Veränderung der Lipidzusammensetzung des Blutes führen; Verbindungen, die die Nahrungsmitteleinnahme oder Nahrungsmittelaufnahme verringern, PPAR - und PXR-Modulatoren und Wirkstoffe, die auf den ATP-abhängigen Kaliumkanal der Betazellen wirken.

Bei einer Ausführungsform der Erfindung werden die Verbindungen der Formel I in Kombination mit Insulin verabreicht.

Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit einem Biguanid, wie z.B. Metformin, verabreicht.
Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Ausführungsform der Erfindung werden die Verbindungen der Formel I in Kombination mit einem PPARgamma Agonist, wie z.B. Rosiglitazon, Pioglitazon, verabreicht.

Bei einer Ausführungsform werden die Verbindungen der Formel I in Kombination mit Verbindungen mit inhibitorischer Wirkung auf SGLT-1 und/oder 2, wie z.B. in WO 2004/007571, WO 2004/052902, WO 2004/052903 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel 1 in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO 96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin.

Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Die Wirksamkeit der Verbindungen wurde wie folgt getestet:

Präparation von Bürstensaummembran-Vesikeln aus dem Dünndarm von Kaninchen, Ratte und Schwein

Die Präparation von Bürstensaummembran-Vesikeln aus den Darmzellen des Dünndarms erfolgte mit der sog. Mg²⁺-Präzipitationsmethode. Die Mucosa aus dem Dünndarm wurde abgeschabt und in 60 ml eiskaltem Tris/HCl-Puffer (ph 7,1) / 300 mM Mannit, 5 mM EGTA suspendiert. Nach dem Verdünnen auf 300 ml mit eiskaltem destilliertem Wasser wurde mit einem Ultraturrax (18-Stab, IKA Werk Staufen, BRD) 2 x 1 Minute bei 75 % max. Leistung unter Eiskühlung homogenisiert. Nach Zugabe von 3 ml 1 M MgCl₂-Lösung (Endkonzentration 10 mM) lässt man exakt 15 Minuten bei 0° C stehen. Durch die Zugabe von Mg²⁺ aggregieren die Zellmembranen und präzipitieren, mit Ausnahme der Bürstensaummembranen. Nach einer 15-minütigen Zentrifugation bei 3 000 x g (5 000 rpm, SS-34-Rotor) wird der Niederschlag verworfen und der Überstand, der die Bürstensaummembranen enthält, 30 Minuten bei 26 700 x g (15 000 rpm, SS-34-Rotor) zentrifugiert. Der Überstand wird verworfen, der Niederschlag in 60 ml 12 mM Tris/HCl-Puffer (ph 7,1) / 60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubation bei 0°C wird erneut 15 Minuten bei 3 000 x g zentrifugiert. Der Überstand wird anschließend nochmals 30 Minuten bei 46 000 x g (20 000 rpm, SS-34-Rotor) zentrifugiert. Der Niederschlag wird in 30 ml 20 mM Tris/Hepes-Puffer (pH 7,4) /280 mM Mannit aufgenommen und durch 20 Hübe in einem Potter Elveihem Homogenisator bei 1 000 rpm homogen resuspendiert. Nach 30-minütiger Zentrifugation bei 48 000 x g (20 000 rpm, SS-34-Rotor) wurde der Niederschlag in 0,5 bis 2 ml Tris/Hepes-Puffer (pH 7,4) /280 mM Mannit (Endkonzentration 20 mg/ml) aufgenommen und mit Hilfe einer Tuberkulinspritze mit einer 27 Gauge-Nadel resuspendiert.
Die Vesikel wurden entweder unmittelbar nach der Präparation für Markierungs- oder Transportuntersuchungen verwendet oder wurden bei - 196°C in 4 mg Portionen in flüssigem Stickstoff aufbewahrt.
Für die Präparation von Bürstensaummembranvesikeln aus Rattendünndarm wurden 6 bis 10 männliche Wistar-Ratten (Tierzucht Kastengrund, Aventis Pharma), durch zervikale Dislokation getötet, die Dünndärme entnommen und mit kalter isotonischer Kochsalzlösung gespült. Die Därme wurden aufgeschnitten und die Mucosa abgeschabt. Die Aufarbeitung zur Isolierung von Bürstensaummembranen erfolgte wie oben beschrieben. Zur Abtrennung von Cytoskelettanteilen wurden die Bürstensaummembranvesikel aus Rattendünndarm mit KSCN als chaotropem ion behandelt.

Für die Präparation von Bürstensaummembranen aus Kaninchendünndarm wurden Kaninchen durch intravenöse Injektion von 0,5 ml einer wässrigen Lösung von 2,5 mg Tetracain-HCl, 100 mg m-Butramid und 25 mg Mebezoniumjodid getötet. Die Dünndärme wurden entnommen, mit eiskalter physiologischer Kochsalzlösung gespült und in Kunststoffbeutel unter Stickstoff bei - 80 °C eingefroren und 4 bis 12 Wochen gelagert. Zur Präparation der Membranvesikel wurden die eingefrorenen Därme bei 30°C im Wasserbad aufgetaut und anschließend die Mucosa abgeschabt. Die Aufarbeitung zu Membranvesikeln erfolgte wie oben beschrieben.

Zur Präparation von Bürstensaummembranvesikeln aus Schweinedarm wurden Jejunumsegmente eines frisch geschlachteten Schweines mit eiskalter isotonischer Kochsalzlösung gespült und in Plastikbeuteln unter Stickstoff bei - 80°C eingefroren. Die Präparation der Membranvesikel erfolgte wie oben beschrieben.

### Messung der Glukoseaufnahme durch Bürstensaummembranvesikel

Die Aufnahme von [¹⁴C]-markierter Glukose in Bürstensaummembranvesikel wurde mittels der Membranfiltrationsmethode gemessen. 10 µl der Bürstensaummembranvesikelsuspension in 10 mM Tris/Hepes-Puffer (pH 7.4)/300 mM Mannitol wurden bei 20°C zu 90 µl einer Lösung von 10 pM [¹⁴C]D-Glukose und den entsprechenden Konzentrationen der betreffenden Hemmstoffe (5-200 µM) in 10 mM Tris/Hepes-Puffer (pH 7.4)/ 100 mM NaCl/100 mM gegeben.
Nach 15 sec. Inkubation wurde der Transportprozess durch Zugabe von 1 ml eiskalter Stopplösung (10 mM Tris/Hepes-Puffer (pH 7.4)/ 150 mM KCI) angehalten und die Vesikelsuspension wurde sofort bei einem Vakuum von 25 bis 35 mbar über ein Membranfilter aus Cellulosenitrat (0,45 µm, 25 mm Durchmesser, Schleicher & Schüll) abgesaugt. Der Filter wurde mit 5 ml eiskalter Stopplösung nachgewaschen.
Jeder Messpunkt wurde als Doppel- oder Dreifachbestimmung ausgeführt.
Zur Messung der Aufnahme radioaktiv markierter Substrate wurde der Membranfilter in 4 ml eines entsprechenden Szintillators (Quickszint 361, Zinsser Analytik GmbH, Frankfurt am Main) aufgelöst und die Radioaktivität durch Flüssigkeisszintillationsmessung bestimmt. Die gemessenen Werte wurden nach Eichung des Gerätes mit Hilfe von Standardproben und nach Korrektur evtl. vorhandener Chemilumiszenz als dpm (Decompositions per minute) erhalten.

Der Aktivitätsvergleich der Wirkstoffe wird anhand von IC50 Daten durchgeführt, die im Transport-Assay an Dünndarm -Bürstensaummembranvesikeln des Kaninchens für ausgewählte Substanzen erhalten wurden (Die Absolutwerte können Spezies- uns Versuchsabhängig sein).

Eine weitere Methode zur Testung der Wirksamkeit der Verbindungen ist die Hemmung der Transportaktivität des humanen natrium-abhängigen Glukosetransporters 1 (SGLT1, SLC5A1) *in vitro:*

### 1. Klonierung eines Expressionsvektors für humanes SGLT1

Die cDNA für humanes SGLT1 wurde über molekularbiologische Standardmethoden wie in Sambrook et al. beschrieben (Sambrook et al., Molecular Cloning, A Laboratory Manual, 2nd Edition), in den pcDNA4/TO Vektor (Invitrogen) eingebracht. Die anschließende Sequenzierung des Inserts ergab vollständige Identität mit den Basen 11 bis 2005 der von Hediger et al. beschriebenen (Hediger et al., Proc. Natl. Acad. Sci. USA 1989, 86, 5748-5752.) und in der GenBank Sequenzdatenbank hinterlegten Basensequenz für humanes SGLT1 (GenBank Accesion Nummer: M24847). Die Basen 11 bis 2005 entsprechen der kompletten kodierenden Region des humanen SGLT1.

### 2. Herstellung einer rekombinanten Zelllinie mit induzierbarer Expression von humanem SGLT1

Der Expressionsvektor für humanes SGLT1 wurde mittels FuGene6-Lipofektion (Roche) in CHO-TRex Zellen (Invitrogen) eingeführt. Zur Selektion von Einzelzellklonen wurde dem Zellkulturmedium (Nutrient Mixture F-12 (Ham), (Invitrogen) supplementiert mit 10% fötalem Kälberserum (BD Biosciences), 10µg/ml Blasticidin S (CN Biosciences), 100 Einheiten/ml Penicillin, 100 Einheiten/ml Streptomycin) 600µg/ml Zeocin (Invitrogen) zugesetzt. Die Funktionalität der aus der Selektion resultierenden Einzelzellklone wurde über deren Aufnahmeaktivität für radioaktiv markiertes Methyl-α -D-Glukopyranosid getestet. Derjenige Zellklon mit der höchsten Aufnahmeaktivität für Methyl-α -D-Glukopyranosid, nachfolgend CHO-TRex-hSGLT1 bezeichnet, wurde für die weiteren Experimente ausgewählt und weiterhin in Anwesenheit von 600µg/ml Zeocin kultiviert.

### 3. Messung der hemmenden Wirkung von Testsubstanzen auf die Aufnahme von Methyl-α -D-Glukopyranosid (α-MDG)

CHO-TRex-hSGLT1 Zellen wurden in einer Konzentration von 50000 Zellen pro Loch in Cytostar-T Scintillating 96-Loch Platten (Amersham Biosciences) in Zellkulturmedium ausgesät und für 24 h kultiviert. Die Expression des rekombinanten humanen SGLT1 wurde durch Zugabe von 1 µg/ml Tetrazyklin für weitere 24 h induziert. Für α-MDG-Aufnahmeexperimente wurden die Zellen mit PBS gewaschen und anschließend für eine Stunde in Hungermedium (PBS supplementiert mit 10% fötalem Kälberserum) bei 37°C gehungert. Nach einem weiteren Waschschritt mit Transport Assay Puffer (140mM Natriumchlorid, 2mM Kaliumchlorid, 1 mM Magnesiumchlorid, 1 mM Kalziumchlorid, 10mM HEPES/Tris, pH7,5) wurden die Zellen für 15 min bei Raumtemperatur entweder in Abwesenheit oder Anwesenheit von Testsubstanzen unterschiedlicher Konzentration inkubiert. Die Testsubstanzen wurden ausgehend von einer 10mM Stammlösung in Dimethylsulfoxid entsprechend in Transport Assay Puffer verdünnt (40µl/Loch). Das Assay wurde anschließend durch Zugabe von 10µl einer Mischung aus radioaktiv markiertem Methyl-α -D-[U-¹⁴C]Glukopyranosid (Amersham) und unmarkiertem Methyl-α -D-Glukopyranosid (Acros) gestartet. Die Endkonzentration von Methyl-α -D-Glukopyranosid im Assay lag bei 50µM. Nach einer Inkubationszeit von 30 min bei Raumtemperatur wurde die Reaktion durch Zugabe von 50µl/Loch 10mM Methyl-α -D-Glukopyranosid in Transport Assay Puffer (4°C) gestoppt und die in die Zellen aufgenommene Radioaktivität in einem MicroBeta Scintillation Microplate Reader (Wallac) bestimmt. Die halbmaximale Hemmwirkung der Testsubstanzen (IC50 Wert) wurde folgendermaßen bestimmt:
1. Feststellung des Wertes für 0% Inhibition. Dies ist der Messwert bei Abwesenheit von Substanz, gemessen in natrium-haltigem Transport Assay Puffer.
2. Feststellung des Wertes für 100% Inhibition. Dies ist der Messwert bei Abwesenheit von Substanz, gemessen in natrium-freiem Transport Assay Puffer (140mM Cholinchlorid, 2mM Kaliumchlorid, 1 mM Magnesiumchlorid, 1mM Kalziumchlorid, 10mM HEPES/Tris, pH7,5).
3. Errechnung der prozentualen Hemmwerte derjenigen Messungen, die in Anwesenheit unterschiedlicher Konzentrationen von Testsubstanz durchgeführt wurden. Daraus konnte dann diejenige Konzentration der Testsubstanz ermittelt werden, welche die Aufnahme des Methyl-α -D-Glukopyranosids um 50% reduziert (IC50 Wert).
IC50 Werte von Testsubstanzen (µM)

### [in vitro Testung der Aufnahme von Methyl-α -D-Glukopyranosid]

| Beispiel Nr. | IC₅₀ [µM] |
|---|---|
| 3 | 0,043 |
| 6 | 0,133 |
| 9 | 0,081 |
| 12 | 0,139 |
| 15 | 0,170 |
| 18 | 0,080 |
| 21 | 0,047 |
| 22 | 0,144 |
| 24 | 0,208 |
| 31 | 0,252 |
| 33 | 0,070 |
| 36 | 0,043 |

Die nachfolgend aufgeführten Beispiele dienen zur Erläuterung der Erfindung, ohne diese jedoch einzuschränken.

**Tabelle 1: Verbindungen der Formel I**

| | | | | | | | | | | | | | | | |
|---|---|---|---|---|---|---|---|---|---|---|---|---|---|---|---|
| 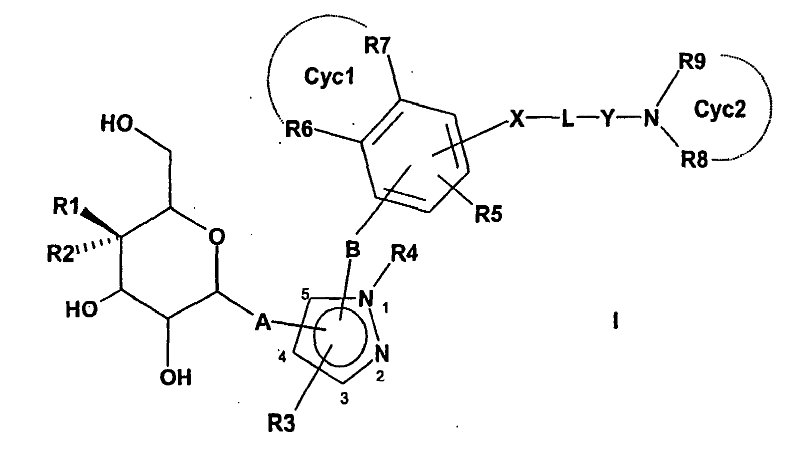 | | | | | | | | | | | | | | | |

| **Bsp** | **R1** | **R2** | **R3** | **R4** | **R5** | **R6** | **R7** | **R8, R9** | **A** | **B** | **X** | **L** | **Y** | **MS*** | **t_{R}[min]** |
|---|---|---|---|---|---|---|---|---|---|---|---|---|---|---|---|
| 1 | H | F | i-Pr | H | H | H | H | CH₂CH₂NHCH₂CH₂ | O | CH₂ | - | -CH=CHCH₂- | - | 505,47 | 1,19 |
| 2 | H | F | i-Pr | H | H | H | H | H; CH₂CH₂CH₂CH₃ | O | CH₂ | - | -CH₂CH₂CH₂- | CO | 522,57 | 1,74 |
| 3 | H | F | i-Pr | H | H | H | H | H; CH₂CH₂CONH₂ | O | CH₂ | - | -CH=CHCH₂- | CO | 535,44 | 1,15 |
| 4 | H | F | i-Pr | H | H | H | H | H; CH₂CH₂CONH₂ | O | CH₂ | - | -CH₂CH₂CH₂- | CO | 537,44 | 1,13 |
| 5 | H | F | i-Pr | H | H | H | H | H; CH₂CONH₂ | O | CH₂ | - | -CH₂CH₂CH₂- | CO | 523,38 | 1,15 |
| 6 | H | F | CH₃ | H | H | H | H | H; CH₂CH₂CONH₂ | O | CH₂ | - | -CH₂CH₂CH₂- | CO | 509,33 | 1,02 |
| 7 | H | F | i-Pr | H | H | H | H | H; CH₂CH₂CONH₂ | O | CH₂ | - | -CH₂CH₂- | CO | 523,42 | 1,36 |
| 8 | H | F | i-Pr | H | H | H | H | H; CH₂CONH₂ | O | CH₂ | - | -CH₂CH₂- | CO | 509,29 | 1,08 |
| 9 | H | F | i-Pr | H | H | H | H | H; CH[CH₂OH]CONH₂ | O | CH₂ | - | -CH₂CH₂CH₂- | CO | 552,29 | 1,13 |
| 10 | H | F | CH₃ | H | H | H | H | H; CH[CH₂OH]CONH₂ | O | CH₂ | - | -CH₂CH₂CH₂- | CO | 525,31 | 1,02 |
| 11 | H | F | CH₃ | H | H | H | H | CH₂CH₂N[CH₂CH₂OH]CH₂CH₂ | O | CH₂ | - | -CH₂CH₂CH₂- | CO | 551,30 | 0,95 |
| 12 | H | F | i-Pr | H | H | H | H | CH₂CH₂N[CH₃]CH₂CH₂ | O | CH₂ | - | -CH₂CH₂CH₂- | CO | 549,30 | 1,07 |
| 13 | H | F | i-Pr | H | H | H | H | CH₂CH₂CH₂CH₂CH₂ | O | CH₂ | - | -CH₂CH₂CH₂- | CO | 534,54 | 1,77 |
| 14 | H | F | i-Pr | H | H | H | H | CH₂CH₂CH₂CH₂CH₂CH₂ | O | CH₂ | - | -CH₂CH₂CH₂- | CO | 548,56 | 1,83 |
| 15 | H | F | i-Pr | H | H | H | H | CH₂CH₂CH₂CH₂ | O | CH₂ | - | -CH₂CH₂CH₂- | CO | 520,52 | 1,67 |
| 16 | H | F | i-Pr | H | H | H | H | CH₂CH₂NHCH₂CH₂ | O | CH₂ | - | -CH₂CH₂CH₂- | CO | 535,32 | 1,06 |
| 17 | H | F | i-Pr | H | H | H | H | H; CH₂CH₂OH | O | CH₂ | - | -CH₂CH₂- | CO | 496,43 | 1,37 |
| 18 | H | F | i-Pr | H | H | H | H | H; C[CH_{3]2}CH₂OH | O | CH₂ | - | -CH₂CH₂- | CO | 524,26 | 1,34 |
| 19 | H | F | i-Pr | H | H | H | H | H; C[CH₃]₂CH₂OH | O | CH₂ | - | -CH₂CH₂CH₂- | CO | 538,28 | 1,26 |
| 20 | H | F | i-Pr | H | H | H | H | H; CH₂CH₇OH | O | CH₂ | - | -CH=CH- | CO | 494,28 | 1,10 |
| 21 | H | F | i-Pr | H | H | H | H | H; C[CH₂OH]₃ | O | CH₂ | - | -CH₂CH₂CH₂- | CO | 570,33 | 1,14 |
| 22 | H | F | i-Pr | H | H | H | H | H; CH₂CH₂CH₂NH₂ | O | CH₂ | - | -CH₂CH₂CH₂- | CO | 522,52 | 1,74 |
| 23 | H | F | i-Pr | H | H | H | H | 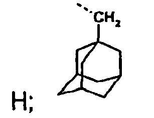 | O | CH₂ | - | -CH₂CH₂CH₂- | CO | 614,45 | 2,11 |
| 24 | H | F | i-Pr | H | H | H | H | 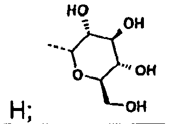 | O | CH₂ | - | -CH₂CH₂CH₂- | CO | 628,25 | 1,07 |
| 25 | H | F | i-Pr | H | H | H | H | CH₂CH₂N[SO₃H]CH₂CH₂ | O | CH₂ | - | -CH₂CH₂CH₂- | CO | 615,42 | 1,64 |
| 26 | H | F | i-Pr | H | H | H | H | H; CH₂CH₂CH₂NHSO₃H | O | CH₂ | - | -CH₂CH₂CH₂- | CO | 603,41 | 1,56 |
| 27 | H | F | i-Pr | H | H | H | H | H; CH₂CH₂OSO₃H | O | CH₂ | - | -CH₂CH₂CH₂- | CO | 588,50 | 1,60 |
| 28 | H | F | i-Pr | H | H | H | H | H; C[CH₃]₂CH₂OSO₃H | O | CH₂ | - | -CH₂CH₂CH₂- | CO | 616,52 | 1,61 |
| 29 | H | F | i-Pr | H | H | H | H | CH₂CH₂OCH₂CH₂ | O | CH₂ | - | -CH₂CH₂CH₂- | CO | 536,48 | 1,58 |
| 30 | H | F | i-Pr | H | H | H | H | H; C[CH₃]₂CH₂CH₃ | O | CH₂ | - | -CH₂CH₂CH₂- | CO | 536,54 | 1,84 |
| 31 | H | F | CH₃ | H | H | H | H | H; CH₂CH₂CH₃ | O | CH₂ | - | -CH₂CH₂CH₂- | NHCO | 494,12 | 2,77 |
| 32 | H | F | i-Pr | H | H | H | H | H; H | O | CH₂ | CO | -NHCH₂CH₂- | - | 494,97 | 0,97 |
| 33 | H | F | i-Pr | H | H | H | H | H; H | O | CH₂ | CO | -NHCH₂- | - | 481,19 | 1,02 |
| 34 | H | F | CF₃ | H | H | H | H | H; H | O | CH₂ | CO | -NHCH₂CH₂- | - | 521,16 | 1,00 |
| 35 | H | F | CF₃ | H | H | H | H | H; H | O | CH₂ | CO | -NHCH₂- | - | 507,16 | 1,20 |
| 36 | H | F | i-Pr | H | H | H | H | CH₂CH₂N[CH₃]CH₂CH₂ | O | CH₂ | - | -CH₂CH₂CH₂- | NHCO | n.d. | n.d. |
| 37 | H | F | i-Pr | H | H | H | H | H; C[CH₃]₂CH₂OH | O | CH₂ | - | -CH₂CH₂CH₂- | NHCO | n.d. | n.d. |

| | | | | | | | | | | | | | | | |
|---|---|---|---|---|---|---|---|---|---|---|---|---|---|---|---|
| Die Verknüpfungen sind in der Beschreibung der Beispiele im Experimentellen Teil angegeben. *Gradient für LCMS: Acetonitril+0,05% TFA:Wasser+0.05% TFA: 5:95 (0 min) zu 95:5 (2,5 min) zu 95:5 (3 min); Säule: YMC J'shere 33x2, 4□M, 1,3 mL/min Flow (Gradient 1). Im experimentellen Teil sind weitere, davon abweichende LCMS-Gradienten angegeben: Gradient 2: 0 min 96%H₂O (0,05% TFA) bis 2,0 min-95% MeCN, dann bis 2.4 min 95% MeCN; dann auf 4% MeCN bis 2,45min.; 1 mL/min; 110-1000MW; 0,4 □L (YMC J'sphere ODS H80 20X21,4 □□□̃□ Gradient 3: 0 min 95%H₂O (5 mmol Ammoniumacetat) bis 3,5 min auf 95% MeCN, dann für 2 min 95% ACN; dann in einer Minute auf 5% MeCN; 0,5mL/min; 115-1000MW; 1 □L (Merck Purospher 3□, 2x55 mm), □̃□ | | | | | | | | | | | | | | | |

Gegenstand der Erfindung sind weiterhin Verfahren zur Herstellung der Verbindungen der allgemeinen Formel I.

Nachfolgend wird die Herstellung der Beispiele detailliert beschrieben. Die erfindungsgemäßen Verbindungen können analog oder entsprechend den in WO 0414932 und WO 0418491 beschriebenen Verfahren erhalten werden.

### Experimenteller Teil:

### Reaktions-Schema: Synthese des □-Bromglykosids 4

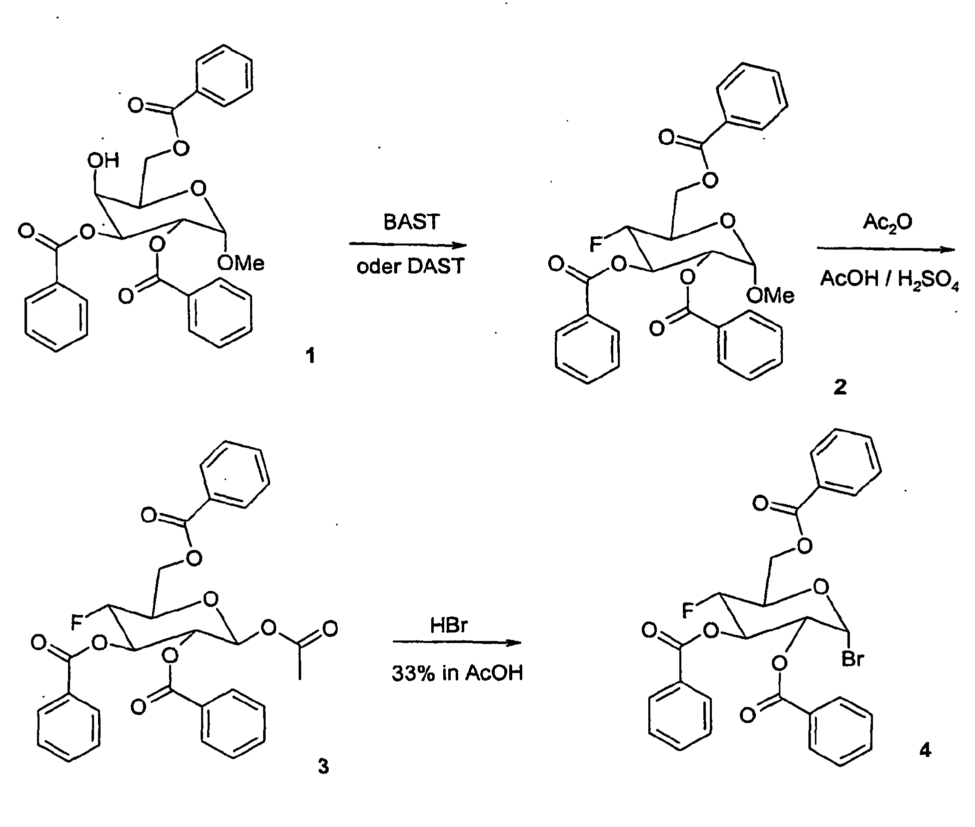

### Methyl-2,3,6-tri-O-benzoyl-4-fluoro-4-deoxy-D-glucopyranosid (2)

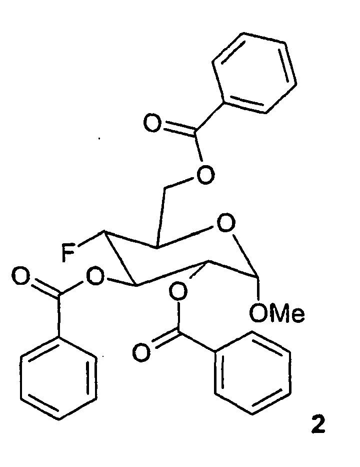

3,0 g Methyl 2,3,6-Tri-O-benzoyl-α-D-galactopyranosid (Reist et al., J.Org.Chem 1965, . 30, 2312) werden in Dichlormethan vorgelegt und auf-30°C gekühlt. Dann werden 3,06 ml [Bis(2-methoxyethyl)amino]sulfurtriflourid (BAST) zugetropft. Die Reaktionslösung wird auf Raumtemperatur erwärmt und 12 h gerührt. Der Ansatz wird mit Dichlormethan verdünnt und die organische Phase mit H₂O, NaHCO₃-Lsg. und gesättigter NaCl-Lsg. extrahiert. Die organische Phase wird über Na₂SO₄ getrocknet und eingeengt. Das Rohprodukt wird aus Ethylacetat und Heptan kristallisiert. Man erhält 1,95g des Produkts **2** als farblosen Feststoff. C₂₈H₂₅FO₈ (508.51) MS (ESI⁺) 526.18 (M+NH₄⁺). Alternativ kann die Reaktion auch unter Verwendung von 2,8 eq. Diethylaminosulfurtrifluorid (DAST) durchgeführt werden; hierbei wird die Reaktionslösung nach erfolgter Zugabe für 18 h refluxiert. Die Aufarbeitung erfolgt analog zu der oben beschriebenen.

### 1-O-Acetyl, 2,3,6-tri-O-benzoyl- 4-fluoro-4-deoxy-glucose (3)

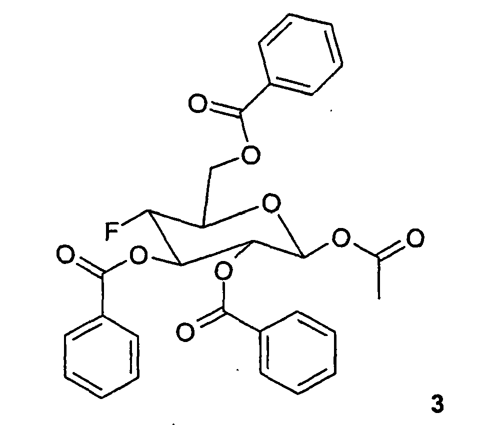

12,0 g Verbindung Methyl-2,3,6-tri-O-benzoyl-4-fluoro-4-deoxy-α-D-glucopyranosid werden in 150 ml Essigsäureanhydrid suspendiert. 8,4 ml konz. Schwefelsäure werden mit 150 ml Eisessig gemischt und unter Eiskühlung zum Ansatz gegeben. Der Ansatz rührt bei Raumtemperatur für 60 h. Das Reaktionsgemisch wird in NaHCO₃-Lsg. gegossen und diese Lösung mit Dichlormethan extrahiert. Die organische Phase wird mit NaCl-Lsg. gewaschen, mit Na₂SO₄ getrocknet und eingeengt. Der Rückstand wird aus Ethylacetat und Heptan umkristallisiert. Man erhält 5,97 g des Produkts 3 als farblosen Feststoff.
C₂₉H₂₅FO₉ (536.52) MS (ESI⁺) 554.15 (M⁺NH₄⁺).

### 1-Bromo-4-deoxy-4-fluoro-2,3,6-tri-O-benzoyl-alpha-D-glucose (4)

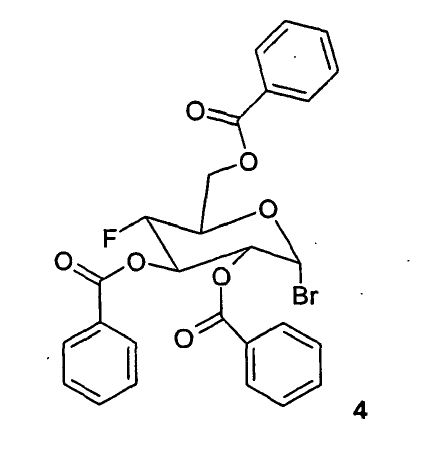

1,44 g 1-*O*-Acetyl,2,3,6-tri-O-benzoyl- 4-fluoro-4-deoxy-glucose werden in 20 ml Bromwasserstoffsäure in Eisessig (33%) gelöst und bei Raumtemperatur gerührt. Nach 5 Stunden wird der Ansatz auf Eiswasser gegeben, die wässrige Phase wird dreimal mit Dichlormethan extrahiert. Die gesammelte organische Phase wird mit gesättigter Natriumchloridlösung gewaschen, über Natriumsulfat getrocknet und zur Trockne eingeengt. Das Rohprodukt wird mit Ethylacetat/Heptan (70:30) über Kieselgel filtriert. Man erhält 1,40 g des Produkts **4** als farblosen Feststoff. C₂₇H₂₂BrFO₇ (557.37) MS (ESI⁺) 574.05/576.05 (M+NH₄⁺).

### Reaktions-Schema I: Synthese von Beispiel 1:

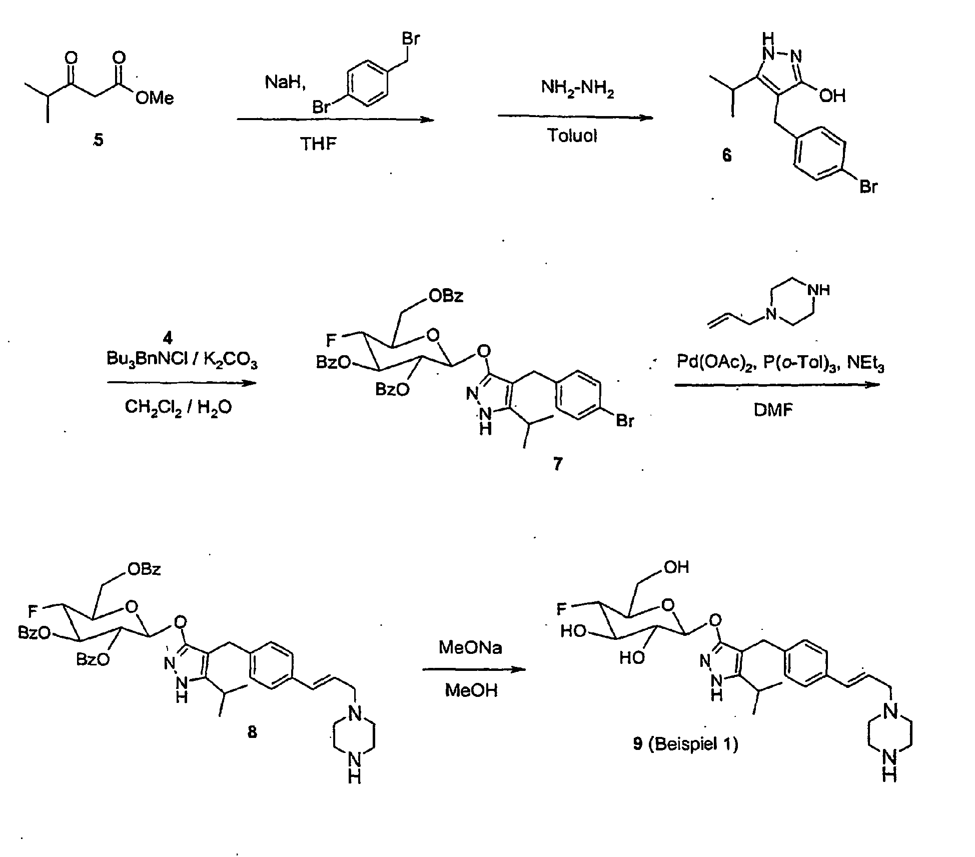

### 4-(4-Brombenzyl)-5-isopropyl-pyraz-3-ol (6):

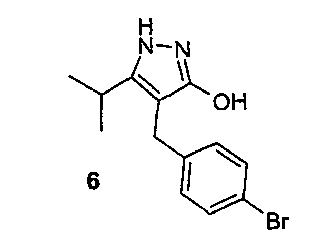

15,2 g Isobutyrylessigsäuremethylester **(5)** werden unter Eiskühlung zu einer Suspension aus Natriumhydrid (60%, 3,85 g) in 250 mL Tetrahydrofuran gegeben. Anschließend wird eine Lösung aus 20,0 g 4-Brombenzylbromid in 100 mL THF zugegeben und 48 h bei Raumtemperatur gerührt. Nach Zugabe von 300 mL H₂O und 300 mL EtOAc, wird die organische Phase über MgSO₄ getrocknet und das Lösemittel wird am Rotationsverdampfer abgezogen. Das erhaltene Rohprodukt wird in 120 mL Toluol gelöst , mit Hydrazin-hydrat (8,01 g) versetzt und 12 h unter Rückfluß am Wasserabscheider erhitzt. Das Reaktiosgemisch wird auf ein Volumen von 50 mL konzentriert und auf 0°C gekühlt. des auskristrallisierten Produkts wird abgesaugt und mit Heptan gewaschen. Man erhält 10,8 g der Verbindung **6** als hellgelben Feststoff. C₁₃H₁₅BrN₂O (295,18) MS (ESI⁺) 294,04 (M+H⁺).

### Verbindung 7:

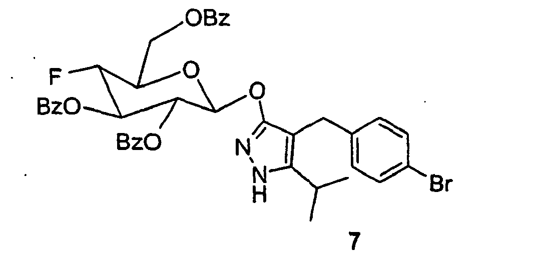

530 mg 4-(4-Brombenzyl)-5-isopropyl-pyraz-3-ol **(6)** und 1.50 g Bromid 4 werden in 50 ml Methylenchlorid gelöst. Zu dieser Lösung werden nacheinander 1.86 g Kaliumcarbonat, 91 mg Benzyltriethylammoniumbromid und 0.8 mL Wasser zugegeben und anschließend 24 Stunden bei Raumtemperatur gerührt. Die Reaktionslösung wird in einen Scheidetrichter überführt und nacheinander mit Wasser und gesättigter Natriumchloridlösung gewaschen. Die organische Phase wird über Magnesiumsulfat getrocknet und am Rotationsverdampfer eingeengt. Das Rohprodukt wird durch Chromatographie über Kieselgel (EtOAc/Heptan) getrennt. Man erhält 193 mg **7** als farblosen Feststoff. C₄₀H₃₆BrFN₂O₈ (771.6) MS (ESI⁺) 773.1 (M+H⁺).

### Verbindung 8:

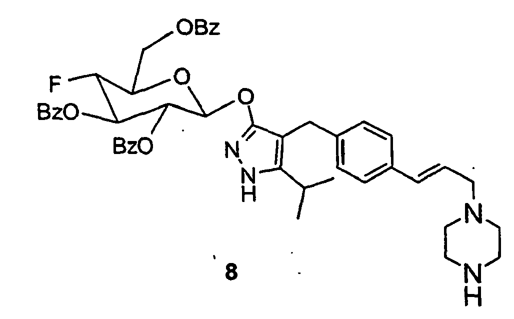

193 mg des Glykosids **7** werden in 1,25 mL DMF gelöst und mit 2,3 mg Pd(OAc)₂, 6,09 mg Tri-o-tolyl-phosphin, 0,25 mL Triethylamin und 84,6 mL 1-Allyl-piperazin versetzt. Das Reaktionsgemisch wird in einem 100°C warmen Ölbad für 18h erhitzt. Das Lösemittel wird am Rotationsverdampfer entfernt und das Rohprodukt wird durch Chromatographie über Kieselgel (EtOAc/MeOH) gereinigt. Man erhält 117 mg der Verbindung **8** als farbloses Wachs. C₄₇H₄₉FN₄O₈ (816,9) MS (ESI⁺) 817,05 (M+H⁺).

### Verbindung 9 (Beispiel 1):

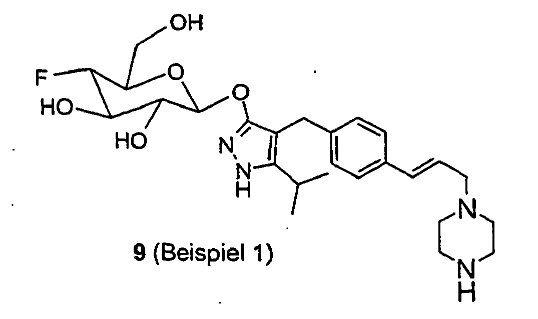

98 mg des Glykosids **8** werden in 4 mL einer Mischung aus Methanol/Wasser/Triethylamin (3:3:1) aufgenommen und 48h bei Raumtemperatur gerührt. Das Reaktionsgemisch wird am Rotationsverdampfer eingeengt und der Rückstand wird durch Chromatographie über Kieselgel (Methylenchlorid/Methanol/konz.Amoniak) gereinigt. Man erhält 34 mg der Verbindung **9** als farblosen Feststoff. C₂₆H₃₇FN₄O₅ (504,61): MS (ESI⁺) 505,47 (M+H⁺).

### Reaktions-Schema II: Synthese von Beispiel 2:

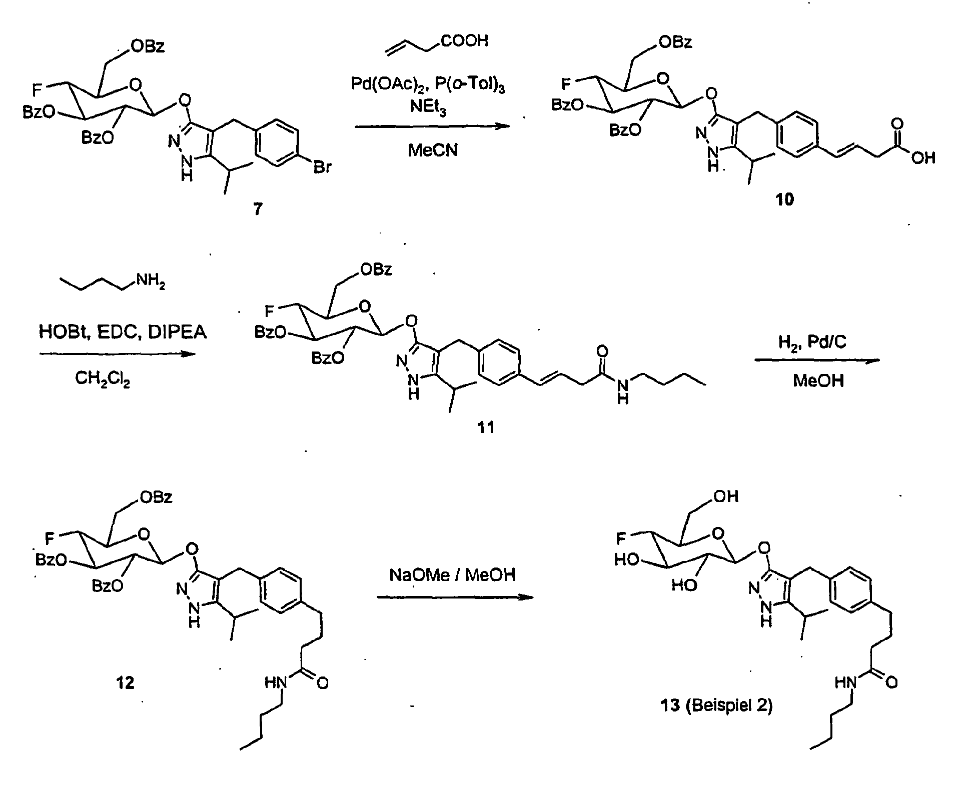

### Verbindung 10:

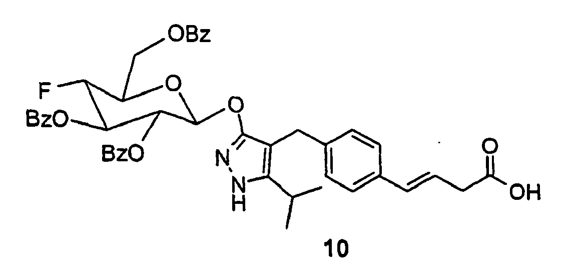

7,20 g des Glykosids **7** werden in 109 mL Acetonitril gelöst und mit 41,9 mg Pd(OAc)₂, 113,6 mg Tri-o-tolyl-phosphin, 39,2 mL Triethylamin und 1,04 g Vinylessigsäure versetzt. Das Reaktionsgemisch wird 60h unter Rückfluß erhitzt. Das Lösemittel wird am Rotationsverdampfer entfernt und das Rohprodukt wird durch Chromatographie über Kieselgel (CH₂Cl₂/MeOH/konz. Ammoniak = 30/5/1) gereinigt. Man erhält 6,18 g der Verbindung **10** als farbloses Wachs. C₄₄H₄₁FN₂O₁₀ (776,8).

### Verbindung 11:

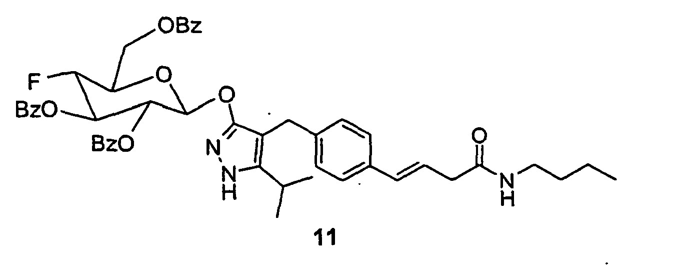

100 mg der Verbindung **10** werden in 4,00 mL Dichlormethan gelöst und 9,41 mg n-Butylamin, 119,8 mg Diisopropylethylamin, 26,1 mg 1-Hydroxybenzotriazol und 30 mg N-(3-Dimethylaminopropyl)N-ethylcarbodiimid werden zugegeben. Das Reaktionsgemisch wird 16h bei 20 °C gerührt. Die Lösung wird nacheinander mit jeweils mit 5 mL NaHCO₃-Lösung, 5 mL 0,2-M-Salzsäure und 5 mL gesättigter NaCL-Lösung gewaschen. Das Lösemittel wird am Rotationsverdampfer entfernt und das Rohprodukt wird ohne weitere Reinigung zur Verbindung **12** umgesetzt. C₄₈H₅₀FN₂₃O₉ (831,9).

### Verbindung 12:

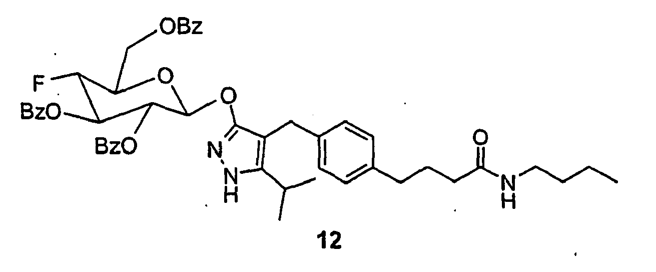

82 mg der Verbindung **11** werden in 5,00 mL Methanol gelöst und 10,5 mg Palladium auf Aktivkohle (10%) werden zugegeben. Das Reaktionsgemisch wird 16h unter 1 bar H₂-Atmosphäre gerührt. Palladium auf Kohle wird abfiltriert, und das Lösemittel wird am Rotationsverdampfer entfernt. Eine weitere Reinigung des Rohprodukts über Kieselgel ist nicht erforderlich. Man erhält 72 mg der gewünschten Verbindung **12** als farbloses Wachs. C₄₈H₅₂FN₂₃O₉ (834,0).

### Verbindung 13 (Beispiel 2):

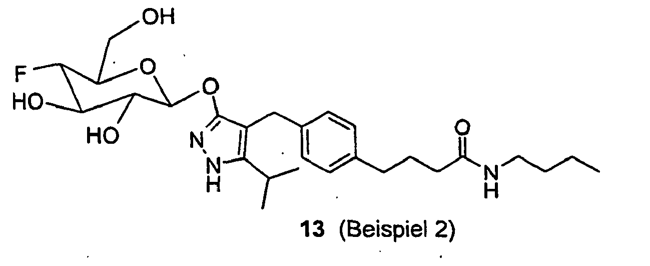

72 mg des Glykosids **12** werden in 10 mL Methanol gelöst und mit 1,72 mL einer 2-M methanolischen Natriummethylat-Lösung versetzt. Das Reaktionsgemisch wird 4h bei 20 °C gerührt und mit 46,2 mg Ammoniumchlorid versetzt. Das Lösemittel wird am Rotationsverdampfer entfernt, und das Rohprodukt wird über Kieselgel gereinigt (zunächst mit Ethylacetat/Heptan = 5/1; anschließend Methylenchlorid/Methanol/konz.Amoniak = 30/5/1). Man erhält 24 mg der Verbindung **13** als farblosen Feststoff. C₂₇H₄₀FN₃O₉ (521,63): MS (ESI⁺) 522,57 (M+H⁺).

### Verbindung 14 (Beispiel 3):

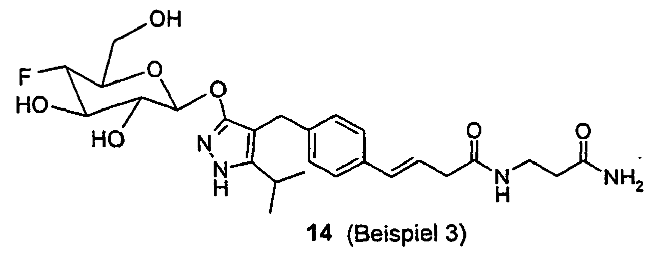

Die Synthese von Verbindung **14** erfolgt analog der beschriebenen Syntheseroute von Verbindung **13** (Beispiel 2). Ausgehend vom Glykosid **10,** das jedoch nicht mit n-Butylamin, sondern mit 3-Aminopropionamid Hydrochlorid umgesetzt wird, und ohne die anschließende Hydrierung durchzuführen, erhält man Verbindung **14** als farblosen Feststoff. C₂₆H₃₅FN₄0₇ (534,6): MS (ESI⁺) 535,44 (M+H⁺).

### Verbindung 15 (Beispiel 4):

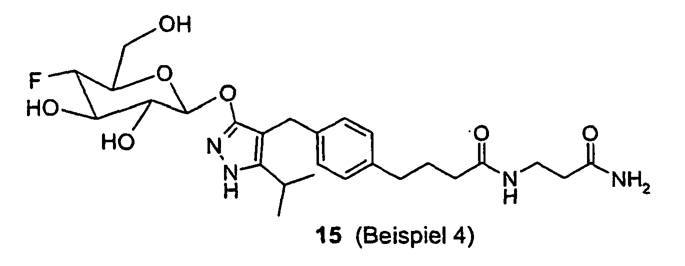

Die Synthese von Verbindung **15** erfolgt analog der beschriebenen Syntheseroute von Verbindung **13** (Beispiel 2). Ausgehend vom Glykosid **10,** das jedoch nicht mit n-Butylamin, sondern mit 3-Aminopropionamid Hydrochlorid umgesetzt wird, erhält man Verbindung **15** als farblosen Feststoff. C₂₆H₃₇FN₄O₇ (536,6): MS (ESI⁺) 537,44 (M+H⁺).

### Verbindung 16 (Beispiel 5):

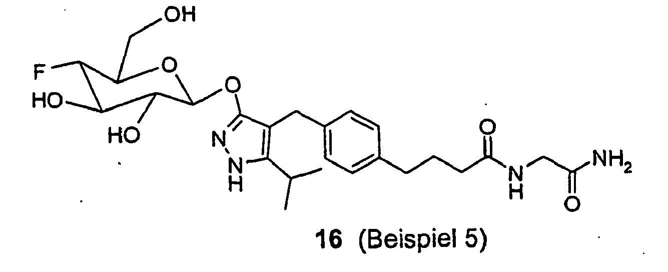

Die Synthese von Verbindung **16** erfolgt analog der beschriebenen Syntheseroute von Verbindung **13** (Beispiel 2). Ausgehend vom Glykosid **10,** das jedoch nicht mit n-Butylamin, sondern mit Glycinamid Hydrochlorid umgesetzt wird, erhält man Verbindung **16** als farbloses Wachs. C₂₅H₃₅FN₄O₇ (522,6): MS (ESI⁺) 523,38 (M+H⁺).

### Verbindung 17:

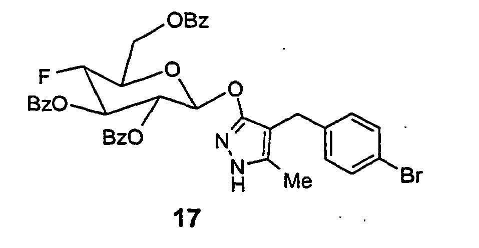

Die Synthese von Verbindung **17** erfolgt analog der beschriebenen Syntheseroute von Verbindung **7** (Schema I). Jedoch wird anstatt von Isobutyrylessigsäuremethylester von Ethylacetoacetat ausgegangen. Man erhält die Verbindung **17** als farblosen Feststoff. C₃₈H₃₂BrFN₂O₈ (743,6).

### Verbindung 18 (Beispiel 6):

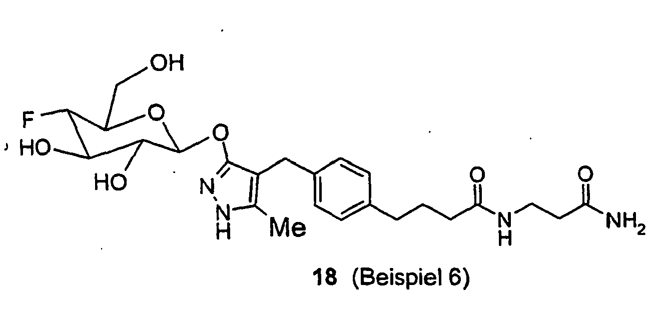

Die Synthese von Verbindung **18** erfolgt analog der beschriebenen Synthese von Verbindung **15** (Beispiel 4). Jedoch wird anstatt von Glykosid **10** von dem Glykosid **17** ausgegangen. Man erhält die Verbindung **18** als farbloses Wachs.
C₂₄H₃₃FN₄O₇ (508,6): MS (ESI⁺) 509,33 (M+H⁺).

### Verbindung 19 (Beispiel 7):

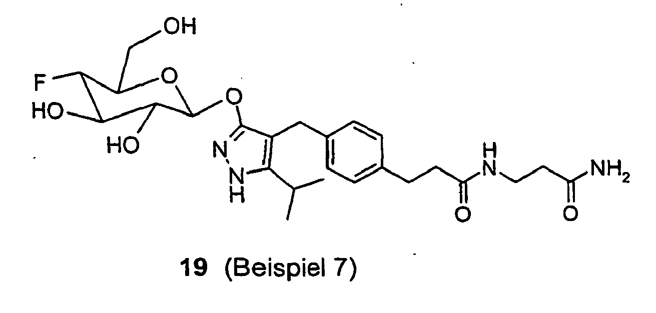

Die Synthese von Verbindung **19** erfolgt analog der beschriebenen Synthese von Verbindung **15** (Beispiel 4). Jedoch wird die Bromverbindung **7** anstatt mit Vinylessigsäure mit Acrylsäure umgesetzt. Man erhält die Verbindung **19** als farbloses Wachs. C₂₅H₃₅FN₄O₇ (522,6): MS (ESI⁺) 523,42 (M+H⁺).

### Verbindung 20 (Beispiel 8):

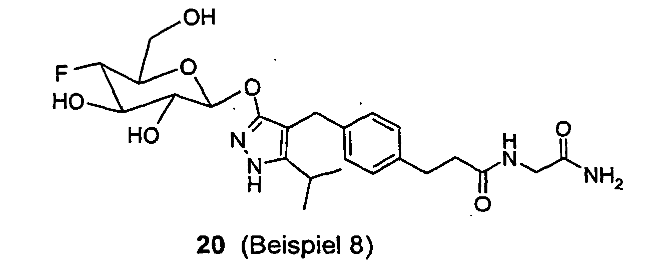

Die Synthese von Verbindung **20** erfolgt analog der beschriebenen Synthese von Verbindung **19** (Beispiel 7). Jedoch wird bei der Amidkupplung 3-Aminopropionamid Hydrochlorid durch Glycinamid Hydrochlorid ersetzt. Man erhält die Verbindung **20** als farbloses Wachs. C₂₄H₃₃FN₄O₇ (508,6): MS (ESI⁺) 509,29 (M+H⁺).

### Verbindung 21 (Beispiel 9):

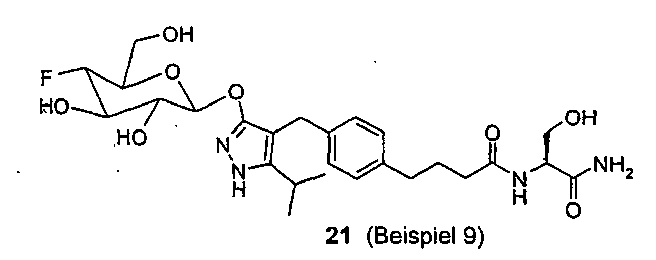

Die Synthese von Verbindung **21** erfolgt analog der beschriebenen Syntheseroute von Verbindung **13** (Beispiel 2). Ausgehend vom Glykosid **10**, das jedoch nicht mit n-Butylamin, sondern mit L-Serinamid Hydrochlorid umgesetzt wird, erhält man Verbindung **21** als farblosen Feststoff. C₂₆H₃₇FN₄O₈ (552,6): MS (ESI⁺) 553,29 (M+H⁺).

### Verbindung 22 (Beispiel 10):

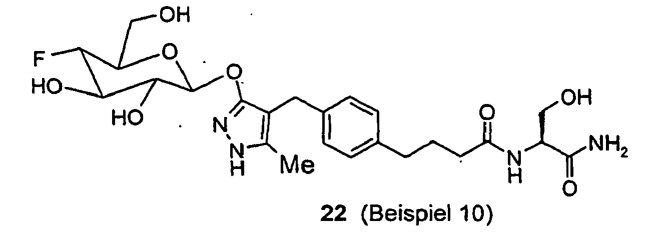

Die Synthese von Verbindung **22** erfolgt analog der beschriebenen Synthese von Verbindung **18** (Beispiel 6). Ausgehend vom Glykosid **17,** das jedoch nicht mit mit 3-Aminopropionamid Hydrochlorid, sondern mit L-Serinamid Hydrochlorid umgesetzt wird, erhält man Verbindung **22** als farbloses Wachs. C₂₄H₃₃FN₄O₈ (524,6): MS (ESI⁺) 525,31 (M+H⁺).

### Verbindung 23 (Beispiel 11):

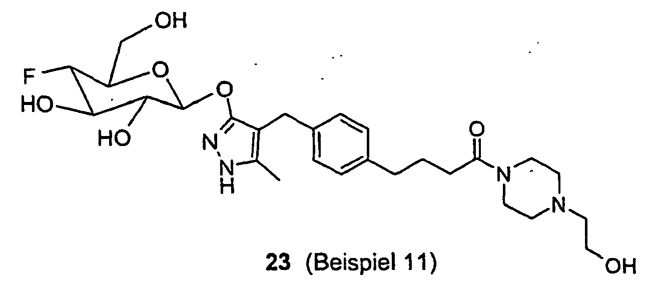

Die Synthese von Verbindung **23** erfolgt analog der beschriebenen Syntheseroute von Verbindung **18** (Beispiel 6). Ausgehend vom Glykosid **17,** das jedoch nicht mit 3-Aminopropionamid Hydrochlorid, sondern mit N-(2-Hydroxyethyl)piperazin umgesetzt wird, erhält man Verbindung **23** als farbloses Wachs. C₂₇H₃₉FN₄O₇ (550,6): MS (ESI⁺) 551,30 (M+H⁺).

### Verbindung 24 (Beispiel 12):

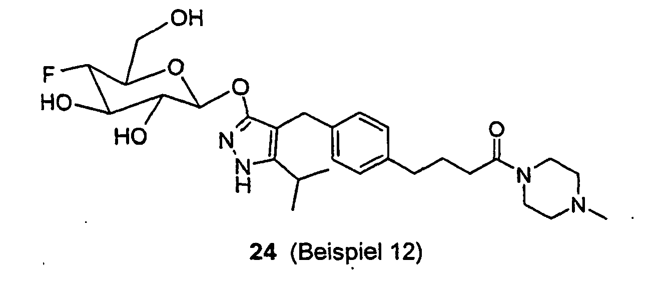

Die Synthese von Verbindung **24** erfolgt analog der beschriebenen Syntheseroute von Verbindung **13** (Beispiel 2). Ausgehend vom Glykosid **10,** das jedoch nicht mit n-Butylamin, sondern mit N-Methylpiperazin umgesetzt wird, erhält man Verbindung **24** als farbloses Wachs. C₂₈H₄₁FN₄O₆ (548,7): MS (ESI⁺) 549,30 (M+H⁺).

### Verbindung 25 (Beispiel 13):

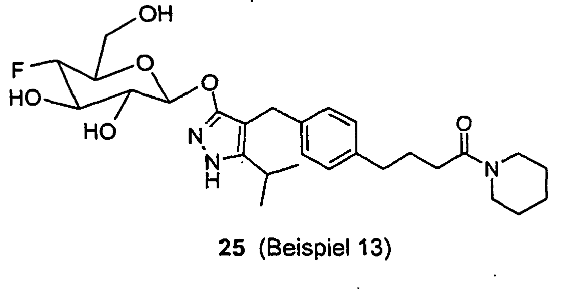

Die Synthese von Verbindung **25** erfolgt analog der beschriebenen Syntheseroute von Verbindung **13** (Beispiel 2). Ausgehend vom Glykosid **10,** das jedoch nicht mit n-Butylamin, sondern mit Piperidin umgesetzt wird, erhält man Verbindung **25** als farbloses Wachs. C₂₈H₄₀FN₃O₆ (533,7): MS (ESI⁺) 534,54 (M+H⁺).

### Verbindung 26 (Beispiel 14):

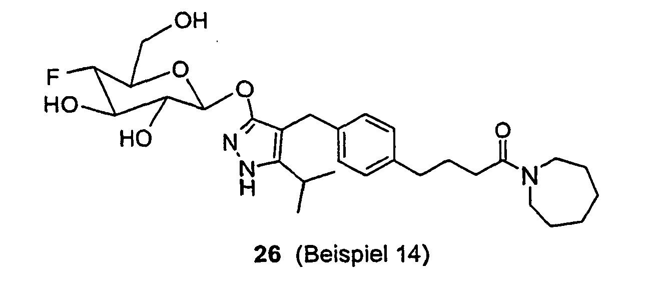

Die Synthese von Verbindung **26** erfolgt analog der beschriebenen Syntheseroute von Verbindung **13** (Beispiel 2). Ausgehend vom Glykosid **10,** das jedoch nicht mit n-Butylamin, sondern mit Hexahydro-1H-azepin umgesetzt wird, erhält man Verbindung **26** als farbloses Wachs. C₂₉H₄₂FN₃O₆ (547,7): MS (ESI⁺) 548,56 (M+H⁺).

### Verbindung 27 (Beispiel 15):

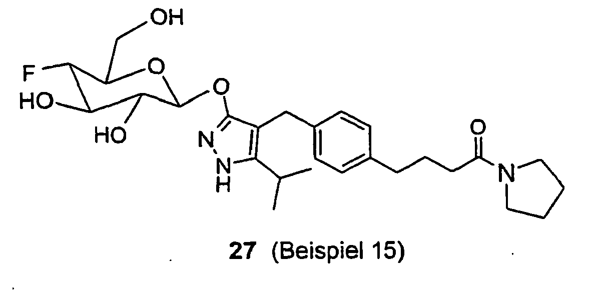

Die Synthese von Verbindung **27** erfolgt analog der beschriebenen Syntheseroute von Verbindung **13** (Beispiel 2). Ausgehend vom Glykosid **10,** das jedoch nicht mit n-Butylamin, sondern mit Pyrrolidin umgesetzt wird, erhält man Verbindung **27** als farbloses Wachs. C₂₇H₃₈FN₃O₆ (519,6): MS (ESI⁺) 520,52 (M+H⁺).

### Verbindung 28 (Beispiel 16):

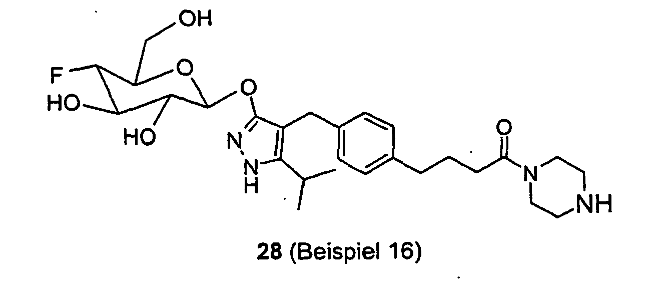

Die Synthese von Verbindung **28** erfolgt analog der beschriebenen Syntheseroute von Verbindung **13** (Beispiel 2). Ausgehend vom Glykosid **10,** das jedoch nicht mit n-Butylamin, sondern mit Benzyl-1-piperazincarboxylat umgesetzt wird, erhält man Verbindung **28** als farbloses Wachs. C₂₇H₃₉FN₄O₆ (534,6): MS (ESI⁺) 535,32 (M+H⁺).

### Verbindung 29 (Beispiel 17):

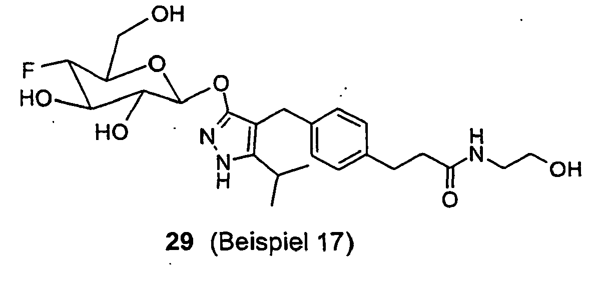

Die Synthese von Verbindung **29** erfolgt analog der beschriebenen Synthese von Verbindung **19** (Beispiel 7). Jedoch wird bei der Amidkupplung 3-Aminopropionamid Hydrochlorid durch 2-Aminoethanol ersetzt. Man erhält die Verbindung **29** als farbloses Öl. C₂₄H₃₄FN₃O₇ (495,6): MS (ESI⁺) 496,43 (M+H⁺):

### Verbindung 30 (Beispiel 18):

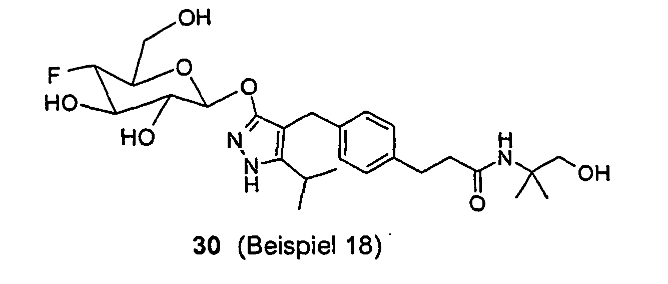

Die Synthese von Verbindung **30** erfolgt analog der beschriebenen Synthese von Verbindung **19** (Beispiel 7). Jedoch wird bei der Amidkupplung 3-Aminopropionamid Hydrochlorid durch 2-Amino-2-methyl-1-propanol ersetzt. Man erhält die Verbindung **30** als farbloses Öl. C₂₆H₃₈FN₃O₇ (523,6): MS (ESI⁺) 524,26 (M+H⁺).

### Verbindung 31 (Beispiel 19):

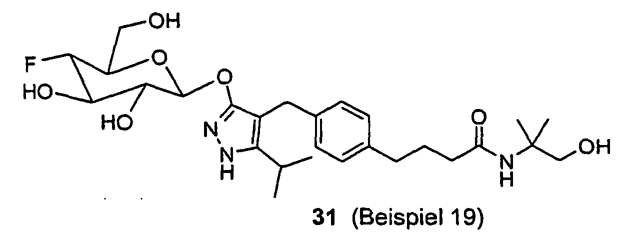

Die Synthese von Verbindung **31** erfolgt analog der beschriebenen Syntheseroute von Verbindung **13** (Beispiel 2). Ausgehend vom Glykosid **10,** das jedoch nicht mit n-Butylamin, sondern mit 2-Amino-2-methyl-1-propanol umgesetzt wird, erhält man Verbindung **31** als farblosen Feststoff. C₂₇H₄₀FN₃O₇ (537,6): MS (ESI⁺) 538,28 (M+H⁺).

### Verbindung 32 (Beispiel 20):

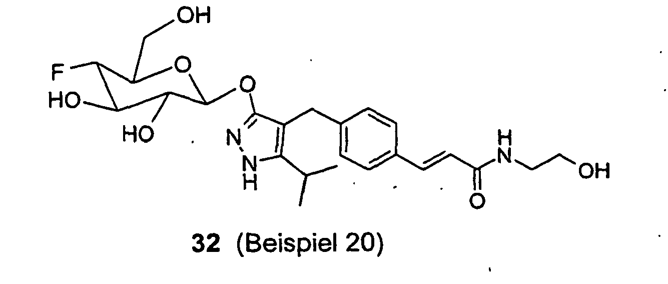

Die Synthese von Verbindung **32** erfolgt analog der beschriebenen Synthese von Verbindung **29** (Beispiel 17). Jedoch wird die Stufe der Hydrierung nicht durchgeführt. Man erhält die Verbindung **32** als farbloses Wachs. C₂₄H₃₂FN₃O₇ (493,6): MS (ESI⁺) 494,28 (M+H⁺).

### Verbindung 33 (Beispiel 21):

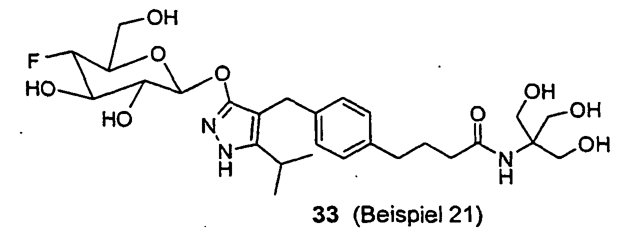

Die Synthese von Verbindung **33** erfolgt analog der beschriebenen Syntheseroute von Verbindung **13** (Beispiel 2). Ausgehend vom Glykosid **10,** das jedoch nicht mit n-Butylamin, sondern mit Tris(hydroxymethyl)aminomethan umgesetzt wird, erhält man Verbindung **33** als farblosen Feststoff. C₂₇H₄₀FN₃O₉ (569,6): MS (ESI⁺) 570,33 (M+H⁺).

### Verbindung 34 (Beispiel 22):

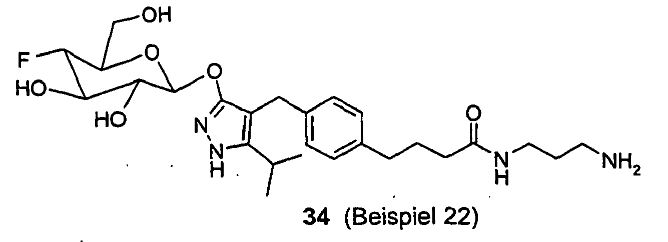

Die Synthese von Verbindung **34** erfolgt analog der beschriebenen Syntheseroute von Verbindung **13** (Beispiel 2). Ausgehend vom Glykosid **10,** das jedoch nicht mit n-Butylamin, sondern mit N-Carbobenzoxy-1,3-diaminopropan Hydrochlorid umgesetzt wird, erhält man Verbindung 34 als farbloses Öl. C₂₆H₃₉FN₄O₆ (522,6): MS (ESI⁺) 522,52 (M+H⁺).

### Verbindung 35 (Beispiel 23):

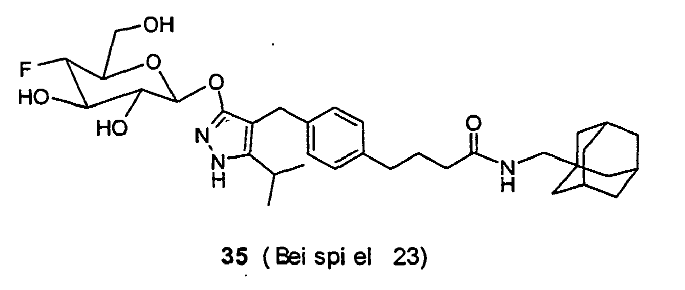

Die Synthese von Verbindung **35** erfolgt analog der beschriebenen Syntheseroute von Verbindung **13** (Beispiel 2). Ausgehend vom Glykosid **10,** das jedoch nicht mit n-Butylamin, sondern mit 1-Adamantan-methylamin umgesetzt wird, erhält man Verbindung **35** als farbloses Wachs. C₃₄H₄₈FN₃O₆ (613,8): MS (ESI⁺) 614,45 (M+H⁺).

### Verbindung 36 (Beispiel 24):

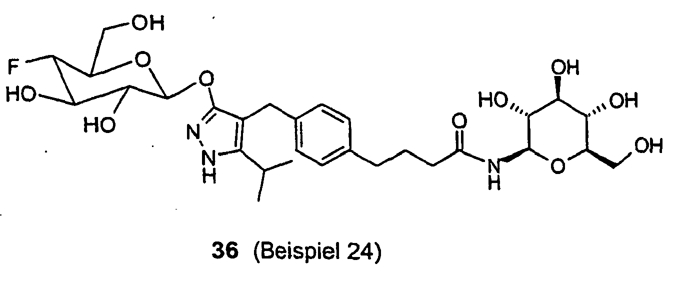

Die Synthese von Verbindung **36** erfolgt analog der beschriebenen Syntheseroute von Verbindung **13** (Beispiel 2). Ausgehend vom Glykosid **10,** das jedoch nicht mit n-Butylamin, sondern mit 2,3,4,6-Tetra-O-acetyl-1-amino-1-deoxy-beta-D-glucose umgesetzt wird, erhält man Verbindung **36** als farbloses Öl. C₂₉H₄₂FN₃O₁₁ (627,7): MS (ESI⁺) 628,25 (M+H⁺).

### Verbindung 37 (Beispiel 25):

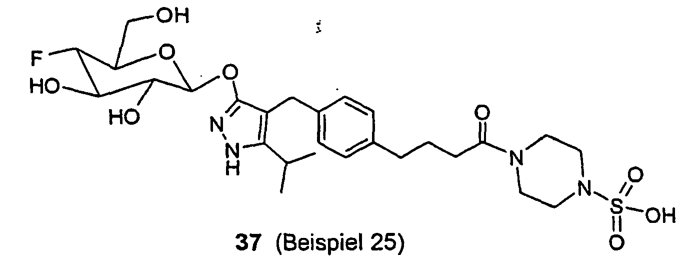

Die Synthese von Verbindung **37** erfolgt analog der beschriebenen Syntheseroute von Verbindung **28** (Beispiel 16). Jedoch wird vor der letzten Stufe, der Entschützung mit Natriummethanolat, eine Umsetzung mit Schwefeltrioxid-Triethylamin Komplex durchgeführt: Dazu werden 63,0 mg der Piperazin-Verbindung in 10,0 mL Methanol gelöst und bei 0 °C mit 202 mg Schwefeltrioxid Triethylamin Komplex versetzt und 2h bei 0 °C gerührt. Das Lösemittel wird am Rotationsverdampfer entfernt, und das Rohprodukt wird über Kieselgel gereinigt (Methylenchlorid/Methanol/konz.Ammoniak = 30/5/1). Man erhält 59 mg der Sulfat-Verbindung, die analog der der Synthese von Verbindung **28** mit Natriummethylat in die Verbindung **37** überführt wird, die man als farbloses Wachs erhält. C₂₇H₃₉FN₄O₉S (614,7): MS (ESI⁺) 615,42 (M+H⁺).

### Verbindung 38 (Beispiel 26):

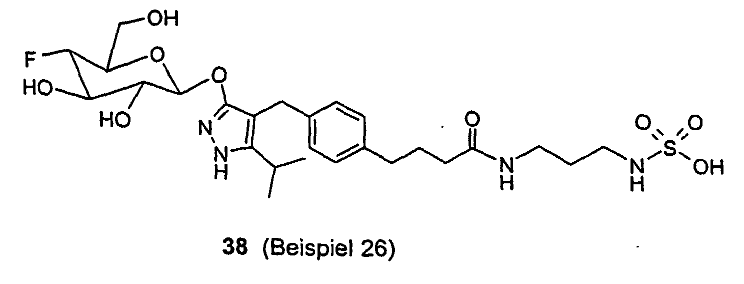

Die Synthese von Verbindung **38** erfolgt analog der beschriebenen Syntheseroute von Verbindung **37** (Beispiel 25). Ausgehend vom Glykosid **10,** das jedoch nicht mit Benzyl-1-piperazincarboxylat, sondern mit N-Carbobenzoxy-1,3-diaminopropan Hydrochlorid umgesetzt wird, erhält man Verbindung 38 als farbloses Wachs. C₂₆H₃₉FN₄O₉S (602,7): MS (ESI⁺) 603,41 (M+H⁺).

### Verbindung 39 (Beispiel 27):

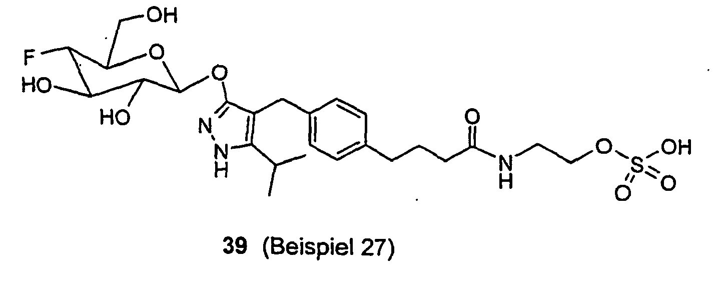

Die Synthese von Verbindung **39** erfolgt analog der beschriebenen Syntheseroute von Verbindung **37** (Beispiel 25). Ausgehend vom Glykosid **10,** das jedoch nicht mit Benzyl-1-piperazincarboxylat, sondern mit 2-Aminoethanol umgesetzt wird, erhält man Verbindung **39** als farbloses Wachs. C₂₅H₃₆FN₃O₁₀S (589,6): MS (ESI⁺) 588,50 (M⁺-H).

### Verbindung 40 (Beispiel 28):

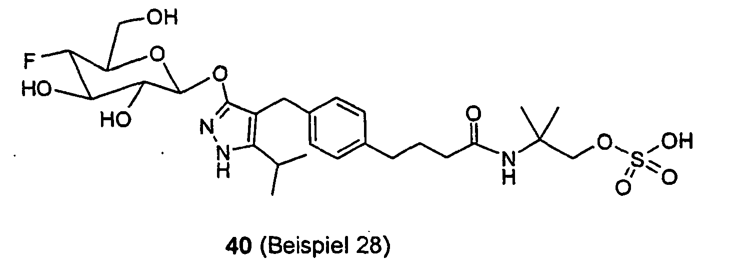

Die Synthese von Verbindung **40** erfolgt analog der beschriebenen Syntheseroute von Verbindung **37** (Beispiel 25). Ausgehend vom Glykosid **10,** das jedoch nicht mit Benzyl-1-piperazincarboxylat, sondern mit 2-Amino-2-methyl-1-propanol umgesetzt wird, erhält man Verbindung **40** als farbloses Wachs. C₂₇H₄₀FN₃O₁₀S (617,7): MS (ESI⁺) 616,52 (M⁺-H).

### Verbindung 41 (Beispiel 29):

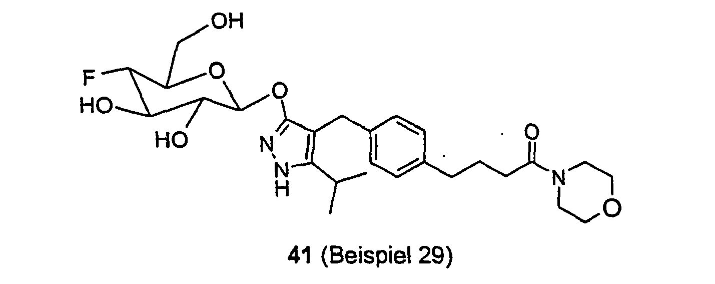

Die Synthese von Verbindung **41** erfolgt analog der beschriebenen Syntheseroute von Verbindung **13** (Beispiel 2). Ausgehend vom Glykosid **10,** das jedoch nicht mit n-Butylamin, sondern mit Morpholin umgesetzt wird, erhält man Verbindung **41** als hellgelbes Wachs. C₂₇H₃₈FN₃O₇ (535,6): MS (ESI⁺) 536,48 (M+H⁺).

### Verbindung 42 (Beispiel 30):

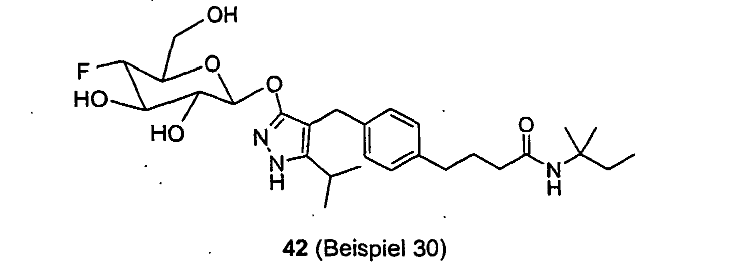

Die Synthese von Verbindung **42** erfolgt analog der beschriebenen Syntheseroute von Verbindung **13** (Beispiel 2). Ausgehend vom Glykosid **10,** das jedoch nicht mit n-Butylamin, sondern mit tert-Amylamin umgesetzt wird, erhält man Verbindung **42** als hellgelbes Wachs. C₂₈H₄₂FN₃O₆ (535,7): MS (ESI⁺) 536,54 (M+H⁺).

### Verbindung 43 (Beispiel 31):

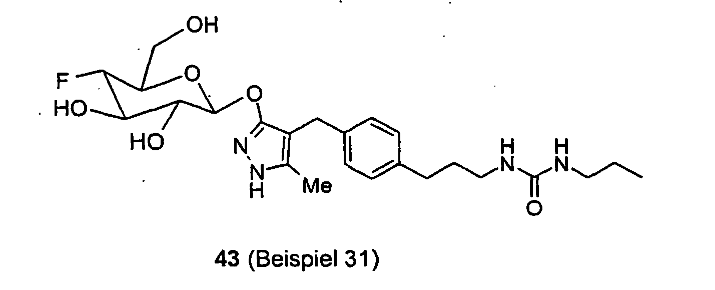

41,3 mg 1-Allyl-3-propyl-harnstoff werden in 5,00 mL THF gelöst und mit 1,21 mL einer 0,5-M 9-BBN-Lösung in Toluol versetzt und 4h bei 20 °C gerührt. Anschließend werden eine Lösung aus 180 mg des Glykosids **17** in 10,0 mL Toluol, 7,4 mg Tri-o-tolyl-phosphin, 102,7 mg Kaliumphosphat und 2,7 mg Pd(OAc)₂ zugegeben. Das Reaktionsgemisch wird 3h auf 100 °C erhitzt. Der Niederschlag wird abfiltriert, und die organische Phase wird mit 10 mL Wasser gewaschen und über Magnesiumsulfat getrocknet. Das Lösemittel wird am Rotationsverdampfer entfernt und das Rohprodukt wird durch Chromatographie über Kieselgel (EtOAc/Heptan) gereinigt. Man erhält 59 mg eines farblosen Feststoffs, der analog der Darstellung von Verbindung 13 (Beispiel 2) mit Natriummethylat umgesetzt wird. Man erhält Verbindung **43** als farbloses Wachs C₂₄H₃₅FN₄O₆ (494,6) MS (ESI⁺) 494,12 (M⁺).

### 4-(2-Ethoxycarbonyl-4-methyl-3-oxo-pent-1-enyl)-benzoesäure (Mischung von E/Z-Isomeren) (44):

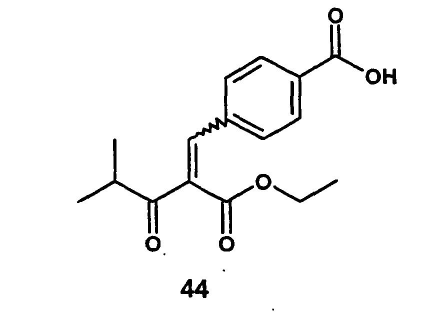

29,0 g Ethylisobutyrylacetat und 33,0 g 4-Carboxybenzaldehyd werden für 6h am Wasserabscheider erhitzt. Die Reaktionslösung wird eingeengt, in Ethylacetat aufgenommen und mit 20%-iger Ammoniumchloridlösung und ges. Natriumchloridlösung extrahiert. Die organische Phase wird über Magnesiumsulfat getrocknet, eingeengt und direkt weiter zu **45** umgesetzt. Man erhält 50,0 g eines Öls. C₁₆H₁₈O₅ (290,3): MS (ESI⁺): 291,1 (M+H)⁺, *t*_{R} = 1,42 min (Gradient 2)

### 4-(2-Ethoxycarbonyl-4-methyl-3-oxo-pentyl)-benzoesäure (45):

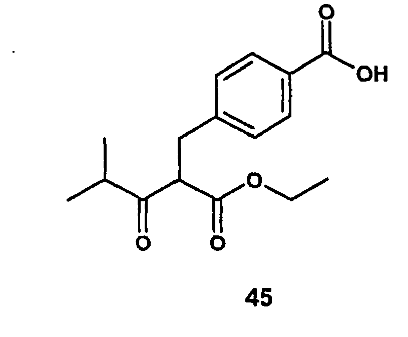

50g 4-(2-Ethoxycarbonyl-4-methyl-3-oxo-pent-1-enyl)-benzoesäure werden in 300 mL THF gelöst, mit 1,00 g Palladium auf Kohle (10%) versetzt und für 24h bei 4bar

Wasserstoffdruck im Autoklaven hydriert. Der Ansatz wird mit Dichlormethan verdünnt, über Celite abgesaugt, der Rückstand wird mit Dichlormethan gewaschen und im Vakuum eingeengt. Der Rückstand wird chromatographisch an Kieselgel gereinigt (Ethylacetat/n-Heptan = 3/1) Man erhält 45 g der Verbindung **45** als Öl. C₁₆H₂₀O₅ (292,3) MS (ESI⁺): 293,1 (M+H)⁺, *t*_{R} = 1,37 min (Gradient 2).

### 4-[1-(2-Cyano-ethyl)-5-hydroxy-3-isopropyl-1H-pyrazol-4-ylmethyl]-benzoesäure (46)

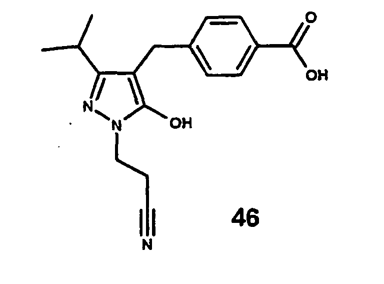

15g der Verbindung **45** werden in 100 mL Eisessig gelöst. 7,4 mL 2-Cyanoethylhydrazin werden zugegeben und die Lösung wird für 2h auf 100°C erhitzt. Der Ansatz wird auf Eiswasser gegeben und mehrfach mit Ethylacetat extrahiert. Die organische Phase wird mit 20%iger Ammoniumchlorid-Lösung und mit ges. Natriumchloridlösung extrahiert und über Natriumsulfat getrocknet. Aus der Ethylacetatphase kristallisieren 2,40 g der gewünschten Verbindung 46. Die Mutterlauge wird eingeengt und an Kieselgel chromatographiert (Dichlormethan:Methanol:Eisessig = 100:10:1). Man erhält weitere 1.10 g der Verbindung **46,** sowie 7,0 g reisoliertes Edukt **45.** C₁₇H₁₉N₃O₃ (313,4); MS (ESI⁺): 314,2 (M+H)⁺, t_{R} = 0,97 min (Gradient 2).

### N-(2-Carbamoyl-ethyl)-4-[1-(2-cyano-ethyl)-5-hydroxy-3-isopropyl-1H-pyrazol-4-ylmethyl]-benzamid (47):

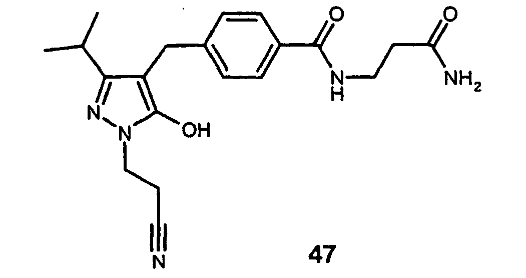

500 mg der Verbindung **46** und 145 mg □-Alaninamid-Hydrochlorid werden in 10 mL Dichlormethan vorgelegt, mit 0,8 mL N,N-Diisopropylethylamin, 215 mg 1-Hydroxybenzotriazol und 306 mg 1-Ethyl-3-(3-Dimethylaminopropyl)carbodiimid Hydrochlorid versetzt. Die Lösung rührt für 12h. Die Lösung wird eingeengt und das Rohprodukt wird über Kieselgel chromatographisch gereinigt (Dichlormethan/Methanol/Eisessig 100:0:5=> 100:10:5). Man erhält 440 mg der gewünschten Verbindung **47.** C₂₀H₂₅N₅O₃ (383,5); MS (ESI⁺): 384,2 (M+H)⁺, t_{R} = 3,58 min (Gradient 3).

### Verbindung 48:

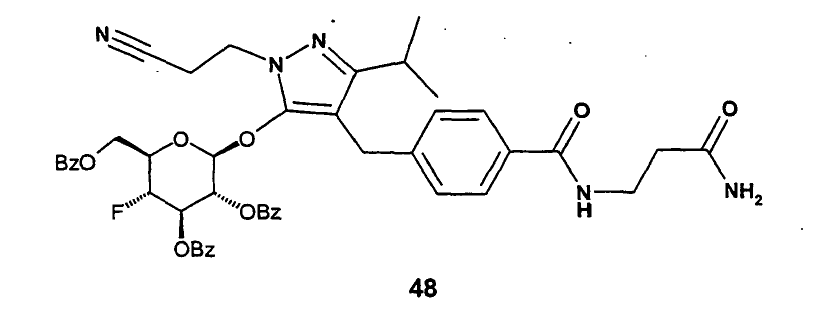

300 mg der Verbindung **47,** 436 mg der Verbindung **4,** und 324 mg Kaliumcarbonat werden in 25 mL Acetonitril und 2,5 mL Wasser suspendiert und für 72h gerührt. Das Reaktionsgemisch wird filtriert, der Rückstand mit Dichlormethan gewaschen und die kombinierte organische Phase mit Wasser und ges. Natriumchloridlösung extrahiert.

Die organische Phase wird über Natriumsulfat getrocknet und der Rückstand an Kieselgel chromatographiert (Dichlormethan/Methanol = 100/5). Man erhält 207 mg des Glykosids **48** als farblosen Feststoff. C₄₇H₄₆FN₅O₁₀ (859,9); MS (ESI⁺): 860,3 (M+H)⁺, *t*_{R}= 1,70 min (Gradient 2).

### Verbindung 49 (Beispiel 32):

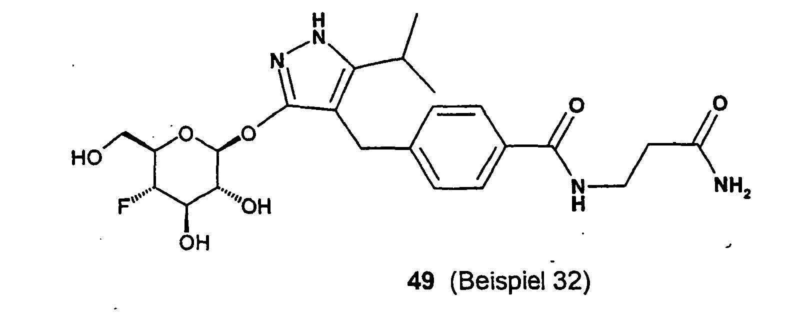

200 mg der Verbindung **48** werden in 15 mL THF gelöst und unter Argon auf-78° gekühlt. Durch ein Septum werden langsam 0,81 mL Lithium-bis(trimethylsilyl)amid-Lösung (1 M in Hexan) zugegeben. Nach 30 min. werden in der Kälte 2 mL 20%-ige Ammoniumchlorid-Lösung zugegeben und die Lösung wird auf Raumtemperatur erwärmt. 2 mL ges. Natriumchloridlösung wird zugegeben, die organische Phase wird abgetrennt und die wässrige Phase wird zweimal mit Ethylacetat extrahiert. Die kombinierte organische Phase wird eingeengt und der Rückstand in ein Gemisch aus Triethylamin : Methanol : Wasser (14 mL 1:3:3) aufgenommen. Die Lösung rührt für 24h, wird anschließend zur Trockene eingeengt und dann über Kieselgel chromatographisch gereinigt. Man erhält 50 mg der Verbindung **49** als farblosen Feststoff. C₂₃H₃₁FN₄O₇ (494,5); MS (ESI⁻): 493,2 (M-H)⁻, t_{R} = 3,58 min (Gradient 3); t_{R} = 0,97 min (Gradient 1).

### Verbindung 50 (Beispiel 33):

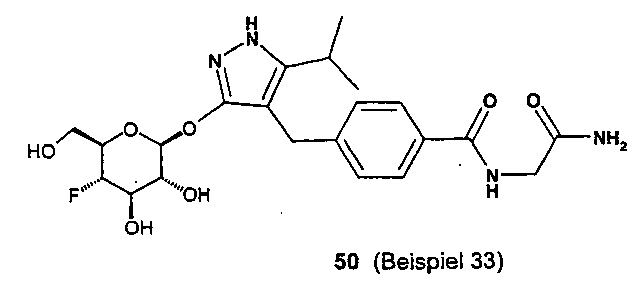

Die Synthese von Verbindung **50** erfolgt analog der beschriebenen Synthese von Verbindung **49** (Beispiel 32). Jedoch wird nicht β-Alaninamid, sondern Glycinamid Hydrochlorid eingesetzt. Man erhält Verbindung **50** als farblosen Feststoff. C₂₂H₂₉FN₄O₇ (480,5): MS (ESI⁺) 481,19 (M+H⁺).

### Verbindung 51 (Beispiel 34)

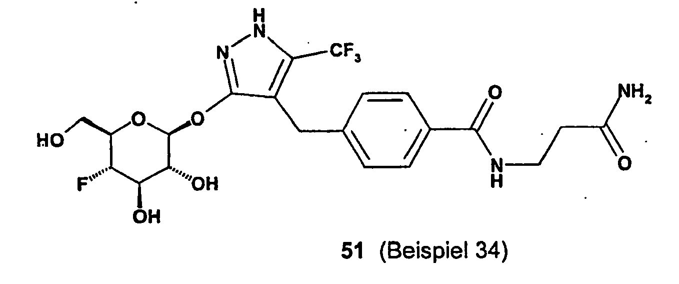

Die Synthese von Verbindung **51** erfolgt analog der beschriebenen Synthese von Verbindung **49** (Beispiel 32). Jedoch wird nicht von Ethyl-isobutylacetat, sondern von 4,4,4-Trifluoracetoacetat ausgegangen. Man erhält Verbindung **51** als farblosen Feststoff. C₂₁H₂₄F₄N₄O₇ (520,4): MS (ESI⁺) 521,16 (M+H⁺).

### Verbindung 52 (Beispiel 35):

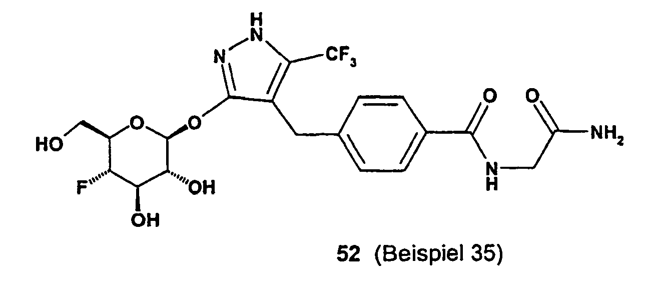

Die Synthese von Verbindung **52** erfolgt analog der beschriebenen Synthese von Verbindung **50** (Beispiel 33). Jedoch wird nicht von Ethyl-isobutylacetat, sondern von 4,4,4-Trifluoracetoacetat ausgegangen. Man erhält Verbindung **52** als farblosen Feststoff. C₂₀H₂₂F₄N₄O₇ (506,4): MS (ESI⁺) 507,16 (M+H⁺).

### Verbindung 53 (Beispiel 36):

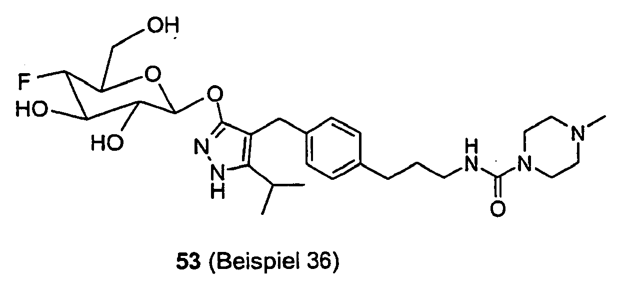

Die Synthese von Verbindung **53** erfolgt analog der beschriebenen Synthese von Verbindung **43** (Beispiel 31). Jedoch wird nicht von 1-Allyl-3-propyl-harnstoff, sondern von 1-(N-Methylpiperazin)-3-Allyl-harnstoff ausgegangen, und statt des Glykosids **17** wird das Glykosid **7** eingesetzt. Man erhält Verbindung **53** als farblosen Feststoff. C₂₈H₄₂FN₅O₆ (563,7).

### Verbindung 54 (Beispiel 37):

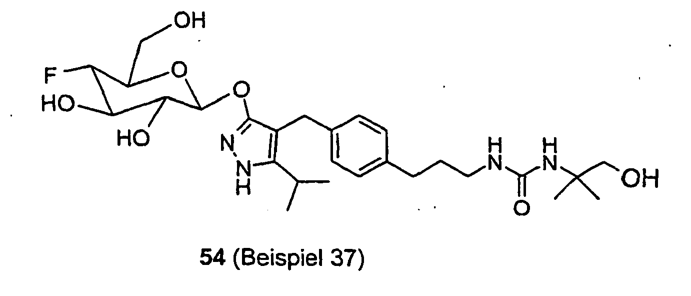

Die Synthese von Verbindung **54** erfolgt analog der beschriebenen Synthese von Verbindung **43** (Beispiel 31). Jedoch wird nicht von 1-Allyl-3-propyl-harnstoff, sondern von 1-(N-Methylpiperazin)-3-Allyl-harnstoff ausgegangen, und statt des Glykosids **17** wird das Glykosid **7** eingesetzt. Man erhält Verbindung **54** als farblosen Feststoff. C₂₇H₄₁FN₄O₇ (552,7).

## Patentansprüche

1. Verbindungen der Formel I, 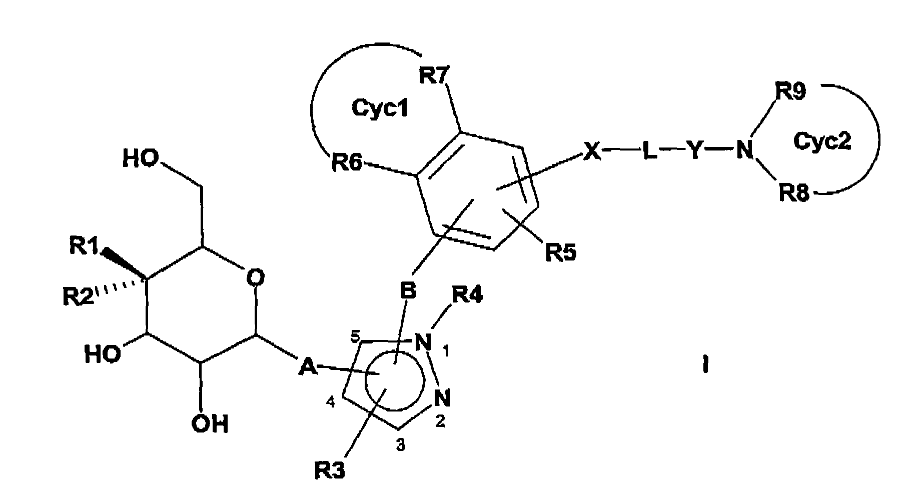 worin bedeuten
R1 und R2 unabhängig voneinander, F oder H, wobei einer der Reste R1 oder R2 F sein muss;
A O, NH, CH₂, S oder eine Bindung;
R3 Wasserstoff, F, Cl, Br, J, OH, CF₃, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁-C₆)-Alkyl, (C₃-C₆)-Cycloalkyl, (C₂-C₆)-Alkenyl, (C₂-C₆)-Alkinyl, O-(C₁-C₆)-Alkyl, HO-(C₁-C₆)-Alkylen, (C₁-C₆)-Alkylen-O-(C₁-C₆)-Alkyl, Phenyl, Benzyl, (C₁-C₆)-Alkoxycarboxyl, wobei in den Alkyl-, Alkenyl-, Alkinyl- bzw. O-Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH-CO-(C₁-C₇)-Alkyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R4 Wasserstoff, (C₁-C₆)-Alkyl, (C₂-C₆)-Alkenyl, (C₃-C₆)-Cycloalkyl, oder Phenyl, dass gegebenenfalls durch Halogen oder (C₁-C₄)-Alkyl substituiert sein kann;
B (C₀-C₁₅)-Alkylen, wobei ein oder mehrere C-Atome des Alkylen-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
R5, R6, R7 unabhängig voneinander Wasserstoff, F, Cl, Br, J, OH, CF₃, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁-C₆)-Alkyl, (C₂-C₆)-Alkenyl, (C₂-C₆)-Alkinyl, O-(C₁-C₈)-Alkyl, HO-(C₁-C₆)-Alkylen, (C₁-C₆)-Alkylen-O-(C₁-C₆)-Alkyl, wobei in den Alkyl-, Alkenyl, Alkinyl bzw. O-Alkylresten ein, mehrere, oder alle Wasserstoff(e) durch Fluor ersetzt sein können;
SO₂-NH₂, SO₂NH(C₁-C₈)-Alkyl, SO₂N[(C₁-C₆)-Alkyl]₂, S-(C₁-G₆)-Alkyl, S-(CH₂)ₒ-Phenyl, SCF₃, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH-CO-(C₁-C₆)-Alkyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, O-(C₁-C₈)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
oder
R6 und R7 gemeinsam mit den sie tragenden C-Atomen ein 5 bis 7 gliedrigen, gesättigter, teilweise oder vollständig ungesättigter Ring Cyc1, wobei 1 oder 2 C-Atom(e) des Ringes auch durch N, O oder S ersetzt sein können und Cyc1 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁-C₄)-Alkyl, OCF₃ substituiert sein kann;
X CO, O, NH, S, SO, SO₂ oder eine Bindung;
L (C₁-C₆)-Alkylen, (C₂-C₅)-Alkenylen, (C₂-C₅)-Alkinylen, wobei jeweils eine oder zwei CH₂-Gruppe(n) durch O oder NH ersetzt sein kann;
Y CO, NHCO, SO, SO₂, oder eine Bindung;
R8, R9 unabhängig voneinander Wasserstoff, SO₃H, Zuckerrest, (C₁-C₆)-Alkyl, wobei ein oder mehrere CH₂-Gruppen des Alkyl-Rests unabhängig voneinander durch (C₁-C₆)-Alkyl, OH, (C₁-C₆)-Alkylen-OH, (C₂₇-C₆)-Alkenylen-OH, O-Zuckerrest, OSO₃H, NH₂, NH-(C₁-C₈)-Alkyl, N[(C₁-C₆)-Alkyl]₂, NH-CO-(C₁-C₆)-Alkyl, NH-Zuckerrest, NH-SO₃H, (C₁-C₆)-Alkylen-NH₂, (C₂-C₆)-Alkenylen-NH₂, (C₀-C₆)-Alkylen-COOH, (C₀-C₆)-Alkylen-CONH₂, (C₀-C₆)-Alkylen-CONH-(C₁-C₆)-Alkyl, (C₀-C₆)-Alkylen-SONH₂. (C₀-C₆)-Alkylen-SONH-(C₁-C₆)-Alkyl, (C₀-C₆)-Alkylen-SO₂NH₂, (C₀-C₆)-Alkylen-SO₂NH-(C₁-C₆)-Alkyl, Adamantyl substituiert sein können;
oder
R8 und R9 gemeinsam mit den sie tragenden N-Atom ein 5 bis 7 gliedrigen, gesättigten Ring Cyc2 bilden, wobei ein oder mehrere CH₂-Gruppen des Ringes auch durch O, S, NH, NSO₃H, N-Zuckerrest, N-(C₁-C₆)-Alkyl, ersetzt sein kann, wobei ein oder mehrere CH₂-Gruppen des Alkyl-Rests unabhängig voneinander durch (C₁-C₆)-Alkyl, OH, (C₁-C₆)-Alkylen-OH, (C₂-C₆)-Alkenylen-OH, NH₂, NH-(C₁-C₆)-Alkyl, N[(C₁-C₆)-Alkyl]₂, NH-CO-(C₁-C₆)-Alkyl, NH-Zuckerrest, (C₁-C₆)-Alkylen-NH₂, (C₂-C₆)-Alkenylen-NH₂, (C₀-C₆)-Alkylen-COOH, (C₀-C₆)-Alkylen-CONH₂, (C₀-C₆)-Alkylen-CONH-(C₁-C₆)-Alkyl, (C₀-C₆)-Alkylen-SONH₂,
(C₀-C₆)-Alkylen-SONH-(C₁-C₆)-Alkyl, (C₀-C₆)-Alkylen-SO₂NH₂, (C₀-C₆)-Alkylen-SO₂NH-(C₁-C₆)-Alkyl substituiert sein können,
sowie deren pharmazeutisch verträgliche Salze.

2. Verbindungen der Formel 1 gemäß Anspruch 1, worin bedeuten
A O, NH, eine Bindung;
R3 Wasserstoff, F, Cl,Br, J, OH, CF₃, NO₂, CN, COOH, CO-(C₁-C₆)-Alkyl, COO-(C₁-C₆)Alkyl, CONH₂, CONH-(C₁-C₆)-Alkyl, CO-N[(C₁-C₆)-Alkyl]₂, (C₁-C₆)-Alkyl, (C₂-C₆)-Alkenyl, (C₂-C₆)-Alkinyl, O-(C₁-C₆)-Alkyl, HO-(C₁-C₆)-Alkylen, (C₁-C₆)-Alkylen-O-(C₁-C₆)-alkyl, Phenyl, Benzyl, wobei in den Alkylresten ein, mehrere, oder alle Wasserstoff(e) durch Fluor ersetzt sein können, SO-(C₁-C₆)Alkyl; oder
R4 Wasserstoff, (C₁-C₆)-Alkyl, (C₂-C₆)-Alkenyl, (C₃-C₆)-Cycloalkyl;
B (C₀-C₆)-Alkylen, wobei ein oder mehrere C-Atom(e) des Alkylen-Rests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkylen)-, -N((C₁-C₆)-Alkylen-Phenylen)- oder -NH- ersetzt sein können.

3. Verbindungen der Formel I gemäß den Ansprüchen 1 oder 2, worin die Zucker-Reste beta(β)-verknüpft sind und die Stereochemie in 2-, 3- und 5-Position des Zuckerrestes D-gluco-konfiguriert ist.

4. Verbindungen der Formel I gemäß Ansprüchen 1 bis 3, worin bedeuten
R1 Wasserstoff und
R2 Fluor;
oder
R1 Fluor und
R2 Wasserstoff;
A O, NH;
R3 Wasserstoff, F, Cl, Br, J, OH, CF₃, (C₁-C₆)-Alkyl, (C₃-C₆)-Cycloalkyl. (C₂-C₆)-Alkenyl, O-(C₁-C₆)-Alkyl, wobei in den Alkylresten ein, mehrere, oder alle Wasserstoff(e) durch Fluor ersetzt sein können;
R4 Wasserstoff, (C₁-C₆)-Alkyl, (C₃-C₆)-Cycloalkyl;
B (C₀-C₄)-Alkylen, wobei ein oder mehrere C-Atom(e) des Alkylen-Rests unabhängig voneinander durch -O-, -(C=O)-, -CH=CH-, - CH(OH)-, -CHF-, -CF₂- oder -NH- ersetzt sein können;
R5, R6, R7 unabhängig voneinander Wasserstoff, F, Cl, Br, J, OH, CF₃, NO₂, CN, COOH, COO(C₁-C₆)-Alkyl, CO(C₁-C₄)-Alkyl, CONH₂, CONH-(C₁-C₆)-Alkyl, CO-N[(C₁-C₆)-Alkyl]₂. (C₁-C₆)-Alkyl. (C₂-C₆)-Alkenyl, (C₂-C₆)-Alkinyl, O-(C₁-C₈)-Alkyl, HO-(C₁-C₆)-Alkylen, (C₁-C₆)-Alkylen-O-(C₁-C₆)-Alkyl, wobei in den Alkyl-., Alkenyl-, Alkinyl- bzw. O-Alkylresten ein, mehrere, oder alle Wasserstoff(e) durch Fluor ersetzt sein können; NH₂, NH-(C₁-C₆)-Alkyl, N((C₁-C₆)-Alkyl)₂, NH-CO-(C₁-C₆)-Alkyl, oder
R6 und R7 gemeinsam mit den sie tragenden C-Atomen ein 5 bis 7 gliedrigen, gesättigter, teilweise oder vollständig ungesättigter Ring Cyc1, wobei 1 oder 2 C-Atom(e) des Ringes auch durch N, O oder S ersetzt sein können und Cyc1 gegebenenfalls durch (C₁-C₆)-Alkyl, (C₂-C₅)-Alkenyl, (C₂-C₅)-Alkinyl, wobei jeweils eine CH₂-Gruppe durch O ersetzt sein kann, oder durch H, F, Cl, OH, CF₃, NO₂, CN, COO(C₁-C₄)-Alkyl, CONH₂, CONH(C₁-C₄)-Alkyl, OCF₃ substituiert sein kann;
X CO, O, NH, eine Bindung;
L (C₁-C₆)-Alkylen, (C₂-C₅)-Alkenylen, wobei jeweils eine oder zwei CH₂-Gruppe(n) durch O oder NH ersetzt sein kann;
Y CO, NHCO, eine Bindung.

5. Verbindungen der Formel I gemäß den Ansprüchen 1 bis 4, worin bedeuten
R1 Wasserstoff;
R2 Fluor;
A O;
R3 CF₃, Methyl, Isopropyl;
R4 Wasserstoff;
B (C₀-C₄)-Alkylen, wobei ein oder mehrere C-Atom(e) des Alkylen-Rests unabhängig voneinander durch -O-, -(C=O)-, -CHF- oder -CF₂- ersetzt sein können;
X CO, O, eine Bindung;
L (C₁-C₄)-Alkylen, (C₂-C₄)-Alkenylen, wobei jeweils eine oder zwei CH₂-Gruppe(n) durch O oder NH ersetzt sein kann;
Y CO, NHCO, eine Bindung.

6. Verbindungen der Formel I gemäß den Ansprüchen 1 bis 5, worin bedeuten
R1 Wasserstoff;
R2 Fluor;
A O;
B -CH₂-;
R5 Wasserstoff, Cl, Methyl, Ethyl, OH, CF₃;
R6, R7 Wasserstoff;
X CO, O, eine Bindung;
L (C₁-C₃)-Alkylen, (C₂-C₃)-Alkenylen, wobei jeweils eine CH₂-Gruppe durch O oder NH ersetzt sein kann;
Y CO, NHCO, eine Bindung.

7. Verbindungen der Formel I gemäß den Ansprüchen 1 bis 6, worin die Substituenten A und B eine benachbarte Stellung (ortho-Stellung) und R3 eine benachbarte Stellung (ortho-Stellung) zu B einnimmt.

8. Verbindungen der Formel I gemäß den Ansprüchen 1 bis 7, worin bedeuten
R8, R9 unabhängig voneinander Wasserstoff, SO₃H, Zuckerrest, (C₁-C₄)-Alkyl, wobei der Alkyl-Rest unabhängig voneinander ein oder mehrfach durch (C₁-C₂)-Alkyl, OH, (C₁-C₂)-Alkylen-OH, OSO₃H, NH₂, CONH₂, SO₂NH₂, NH-SO₃H oder Adamantyl substituiert sein kann;
oder
R8 und R9 gemeinsam mit den sie tragenden N-Atom ein 5 bis 7 gliedrigen, gesättigten Ring Cyc2 bilden, ausgewählt aus der Gruppe Piperazin, welches N-subtituiert sein kann durch (C₁-C₂)-Alkyl, (C₁-C₂)-Alkylen-OH oder SO₃H,
Piperidin, Azepan, Pyrrolidin oder Morpholin.

9. Verbindung, ausgewählt aus der Gruppe: 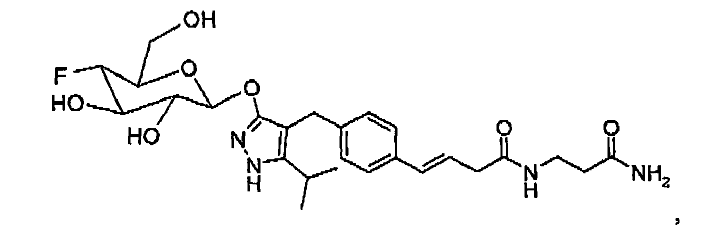 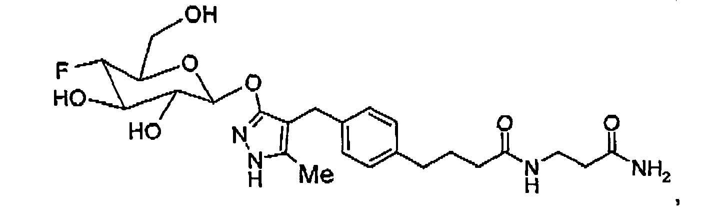 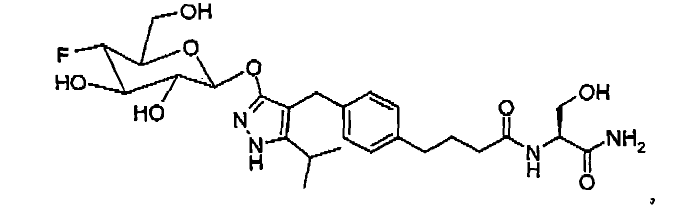 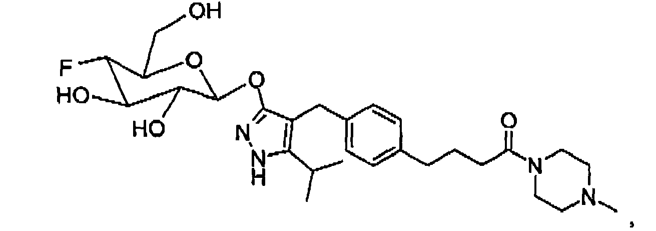 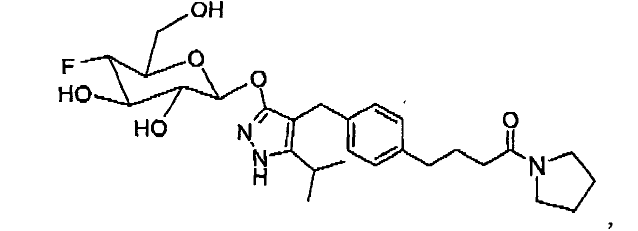 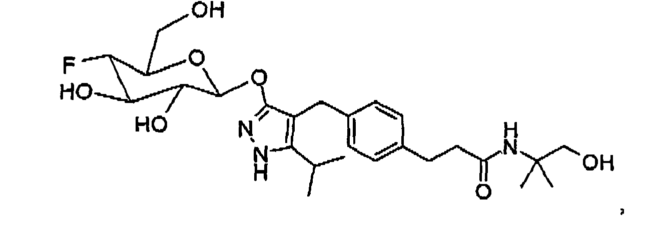 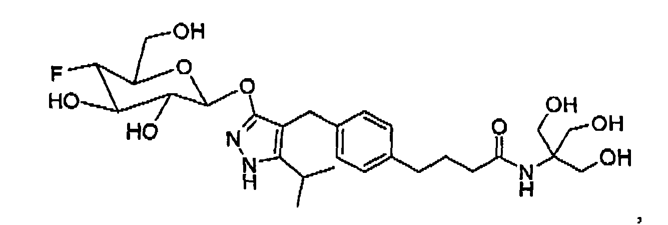 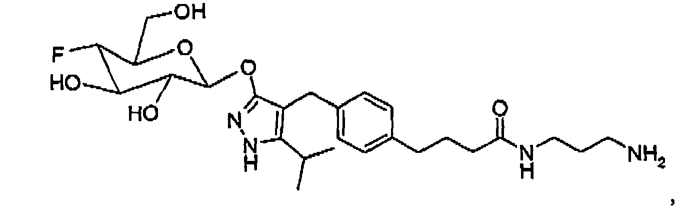 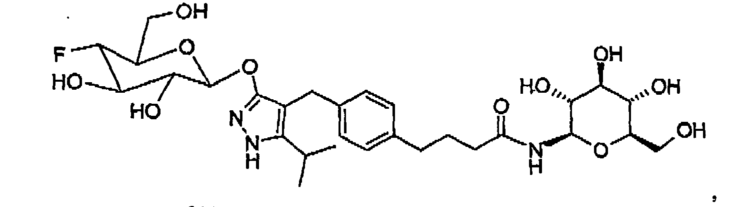 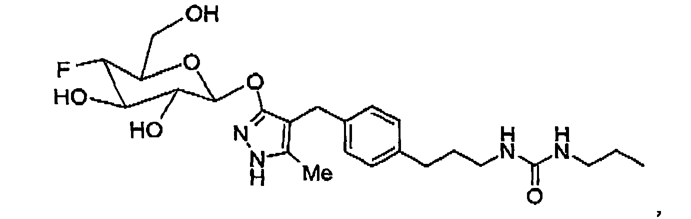 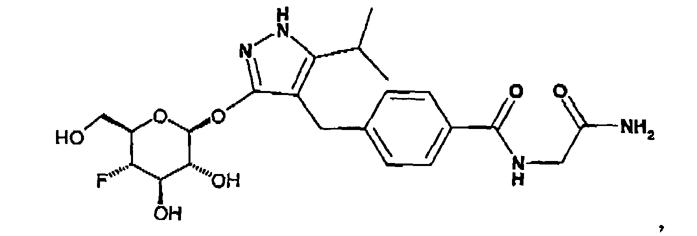 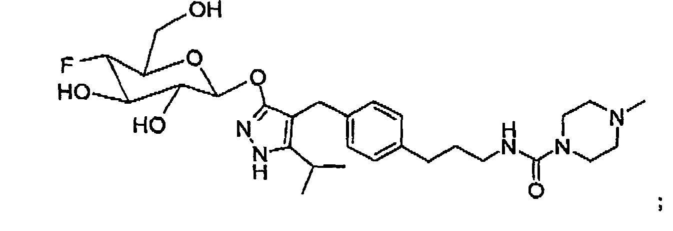 sowie deren pharmazeutisch verträgliche Salze.

10. Arzneimittel enthaltend eine oder mehrere der Verbindungen gemäß den Ansprüchen 1 bis 9.

11. **Arzneimittel enthaltend eine oder mehrere der Verbindungen gemäß den** Ansprüchen 1 bis 9 und ein oder mehrere Blutzucker senkende Wirkstoffe.

12. Verwendung der Verbindungen gemäß den Ansprüchen 1 bis 9 zur Herstellung eines Medikamentes.

13. Verwendung der Verbindungen gemäß den Ansprüchen 1 bis 9 zur Herstellung eines Medikamentes zur Behandlung des Typ 1 und Typ 2 Diabetes.

14. Verwendung der Verbindungen gemäß den Ansprüchen 1 bis 9 zur Herstellung eines Medikamentes zur Blutzuckersenkung.

15. Verwendung der Verbindungen gemäß den Ansprüchen 1 bis 9 in Kombination mit mindestens einem weiteren Blutzucker senkenden Wirkstoff zur Herstellung eines Medikamentes zur Behandlung des Typ 1 und Typ 2 Diabetes.

16. Verwendung der Verbindungen gemäß den Ansprüchen 1 bis 9 in Kombination mit mindestens einem weiteren Blutzucker senkenden Wirkstoff zur Herstellung eines Medikamentes zur Blutzuckersenkung.

17. Verfahren zur Herstellung eines Arzneimittels enthaltend eine oder mehrere der Verbindungen gemäß den Ansprüchen 1 bis 9, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 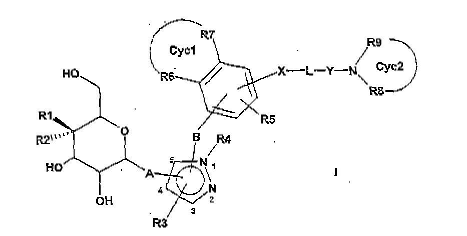 in which the meanings are
R1 and R2 independently of one another F or H, where one of the radicals R1 or R2 must be F;
A 0, NH, CH₂, S or a bond;
R3 hydrogen, F, Cl, Br, I, OH, CF₃, NO₂, CN, COOH, CO-(C₁-C₆)-alkyl, COO(C₁-C₆)-alkyl, CONH₂, CONH-(C₁-C₆)-alkyl, CON[(C₁-C₆)-alkyl]₂, (C₁-C₆)-alkyl, (C₃-C₆)-cycloalkyl, (C₂-C₆)-alkenyl, (C₂-C₆)-alkynyl, O-(C₁-C₆)-alkyl, HO-(C₁-C₆)-alkylene, (C₁-C₆)-alkylene-O-(C₁-C₆)-alkyl, phenyl, benzyl, (C₁-C₆)-alkoxycarboxylic acid, where one, more than one or all hydrogen(s) in the alkyl, alkenyl, alkynyl and O-alkyl radicals may be replaced by fluorine; SO₂-NH₂, SO₂-NH (C₁-C₆)-alkyl, SO₂N [(C₁-C₆)-alkyl]₂, S-(C₁-C₆)-alkyl, S-(CH₂)ₒ-phenyl, SO- (C₁-C₆) -alkyl, SO- (CH₂)ₒ-phenyl, SO₂-(C₁-C₆) -alkyl, SO₂-(CHa)o-phenyl, where o may be 0 - 6, and the phenyl radical may be substituted up to twice by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH-CO-(C₁-C₇)-alkyl, phenyl, O-(CH₂)ₒ-phenyl, where o may be 0 - 6, where the phenyl ring may be substituted one to three times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R4 hydrogen, (C₁-C₆)-alkyl, (C₂-C₆)-alkenyl, (C₃-C₆)-cycloalkyl, or phenyl that may optionally be substituted by halogen or (C₁-C₄) -alkyl;
B (C₀-C₁₅)-alkylene, where one or more C atoms of the alkylene radical may be replaced independently of one another by -O-, -(C=O)-, -CH=CH-, -C≡C-, -S-, -CH(OH)-, -CHF-, -CF₂-, -(S=O)-, (SO₂)-, -N((C₁-C₆)-alkyl)-, -N ((C₁-C₆)-alkylphenyl)- or -NH-;
R5, R6, R7 independently of one another, hydrogen, F, Cl, Br, I, OH, CF₃, NO₂, CN, COOH, COO(C₁-C₆)-alkyl, CO(C₁-C₄)-alkyl, CONH₂, CONH (C₁-C₆) -alkyl, CON [(C₁-C₆)-alkyl]₂, (C₁-C₆)-alkyl, (C₂-C₆)-alkenyl, (C₂-C₆)-alkynyl, O-(C₁-C₆) -alkyl, HO-(C₁-C₆)-alkylene, (C₁-C₆) -alkylene-O-(C₁-C₆)-alkyl, where one, more than one, or all hydrogen(s) in the alkyl, alkenyl, alkynyl and O-alkyl radicals may be replaced by fluorine;
SO₂-NH₂, SO₂NH (C₁-C₆)-alkyl, SO₂N[(C₁-C₆)-alkyl]₂, S-(C₁-C₆)-alkyl, S- (CH₂)ₒ-phenyl, SCF₃, SO-(C₁-C₆)-alkyl, SO-(CH₂)ₒ-phenyl, SO₂(C₁-C₆)-alkyl, SO₂-(CH₂)ₒ-phenyl, where o may be 0 - 6, and the phenyl ring may be substituted up to twice by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH-CO-(C₁-C₆)-alkyl, phenyl, O-(CH₂)ₒ-phenyl, where o may be 0 - 6, where the phenyl ring may be substituted one to three times by F, Cl, Br, I, OH, CF₃, NO₂, CN, OCF₃, O-(C₁-C₈)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
or
R6 and R7 together with the C atoms carrying them a 5 to 7 membered, saturated, partially or completely unsaturated ring Cyc1, where 1 or 2 C atom(s) of the ring may also be replaced by N, 0 or S, and Cyc1 may optionally be substituted by (C₁-C₆)-alkyl, (C₂-C₅) -alkenyl, (C₂-C₅) -alkynyl, where in each case one CH₂ group may be replaced by O, or substituted by H, F, Cl, OH, CF₃, NO₂, CN, COO(C₁-C₄)-alkyl, CONH₂, CONH(C₁-C₄)-alkyl, OCF₃;
X CO, O, NH, S, SO, SO₂ or a bond;
L (C₁-C₆)-alkylene, (C₂-C₅)-alkenylene, (C₂-C₅)-alkynylene, where in each case one or two CH₂ group(s) may be replaced by 0 or NH;
Y CO, NHCO, SO, SO₂, or a bond;
R8, R9 independently of one another, hydrogen, SO₃H, sugar residue, (C₁-C₆)-alkyl, where one or more CH₂ groups of the alkyl radical may be substituted independently of one another by (C₁-C₆)-alkyl, OH, (C₁-C₆)-alkylene-OH, (C₂-C₆)-alkenylene-OH, O-sugar residue, OSO₃H, NH₂, NH-(C₁-C₆)-alkyl, N[(C₁-C₆)-alkyl]₂, NH-CO-(C₁-C₆)-alkyl, NH-sugar residue, NH-SO₃H, (C₁-C₆)-alkylene-NH₂, (C₂-C₆)-alkenylene-NH₂, (C₀-C₆)-alkylene-COOH, (C₀-C₆)-alkylene-CONH₂, (C₀-C₆)-alkylene-CONH-(C₁-C₆)-alkyl, (C₀-C₆ -alkylene-SONH₂, (C₀-C₆)-alkylene-SONH-(C₁-C₆)-alkyl, (C₀-C₆)-alkylene-SO₂NH₂, (C₀-C₆) -alkylene-SO₂NH-(C₁-C₆)-alkyl, adamantyl; or
R8 and R9 together with the N atom carrying them form a 5 to 7 membered, saturated ring Cyc2, where one or more CH₂ groups of the ring may also be replaced by O, S, NH, NSO₃H, N-sugar residue, N-(C₁-C₆)-alkyl, where one or more CH₂ groups of the alkyl radical may be substituted independently of one another by (C₁-C₆)-alkyl, OH, (C₁-C₆)-alkylene-OH, (C₂-C₆)-alkenylene-OH, NH₂, NH-(C₁-C₆)-alkyl, N[(C₁-C₆)-alkyl]₂, NH-CO-(C₂-C₆)-alkyl, NH-sugar residue, (C₁-C₆) -alkylene-NH₂, (C₂-C₆)-alkenylene-NH₂, (C₀-C₆) -alkylene-COOH, (C₀-C₆)-alkylene-CONH₂, (C₀-C₆) -alkylene-CONH- (C₁-C₆)-alkyl, (C₀-C₆) -alkylene-SONH₂, (C₀-C₆)-alkylene-SONH- (C₁-C₆) -alkyl, (C₀-C₆)-alkylene-SO₂NH₂, (C₀-C₆)-alkylene-SO₂NH-(C₁-C₆)-alkyl;
and the pharmaceutically acceptable salts thereof.

2. A compound of the formula I as claimed in claim 1, in which the meanings are
A O, NH, a bond;
R3 hydrogen, F, C1, Br, I, OH, CF₃, NO₂, CN, COOH, CO-(C₁-C₆)-alkyl, COO(C₁-C₆)-alkyl, CONH₂, CONH-(C₁-C₆) -alkyl, CON [(C₁-C₆)-alkyl]₂, (C₁-C₆)-alkyl, (C₂-C₆)-alkenyl, (C₂-C₆) -alkynyl, O-(C₁-C₆)-alkyl, HO-(C₁-C₆) -alkylene, (C₁-C₆ -alkylene-O- (C₁-C₆)-alkyl, phenyl, benzyl, where one, more than one or all hydrogen(s) in the alkyl radicals may be replaced by fluorine, SO-(C₁-C₆)-alkyl; or
R4 hydrogen, (C₁-C₆) -alkyl, (C₂-C₆) -alkenyl, (C₃-C₆)-cycloalkyl;
B (C₀-C₆)-alkylene, where one or more C atom(s) of the alkylene radical may be replaced independently of one another by -O-, -(C=O)-, -CH=CH-, -C≡C-, -S-, -CH(OH)-, -CHF-, -CF₂-, -(S=O)-, -(SO₂)-, -N((C₁-C₆)-alkylene-, -N((C₁-C₆)-alkylene-phenylene)- or -NH-.

3. A compound of the formula I as claimed in claims 1 or 2, in which the sugar residues are beta (13) - linked, and the stereochemistry in the 2, 3 and 5 positions of the sugar residue has the D-gluco configuration.

4. A compound of the formula I as claimed in claims 1 to 3, in which the meanings are
R1 hydrogen and
R2 fluorine;
or
R1 fluorine and
R2 hydrogen;
A O, NH;
R3 hydrogen, F, Cl, Br, I, OH, CF₃, (C₁-C₆)-alkyl, (C₃-C₆)-cycloalkyl, (C₂-C₆)-alkenyl, O-(C₂-C₆)-alkyl, where one, more than one or all hydrogen(s) in the alkyl radicals may be replaced by fluorine;
R4 hydrogen, (C₁-C₆)-alkyl, (C₃-C₆)-cycloalkyl;
B (C₀-C₄) -alkylene, where one or more C atom(s) of the alkylene radical may be replaced independently of one another by -O-, -(C=O)-, -CH=CH-, -CH(OH)-, -CHF-, -CF₂- or -NH-;
R5, R6, R7 independently of one another, hydrogen, F, Cl, Br, I, OH, CF₃, NO₂, CN, COOH, COO(C₁-C₆)-alkyl, CO (C₁-C₄)-alkyl, CONH₂, CONH (C₁-C₆)-alkyl, CON [(C₁-C₆)-alkyl]₂, (C₁-C₆)-alkyl, (C₂-C₆)-alkenyl, (C₂-C₆)-alkynyl, O-(C₁-C₈)-alkyl, HO-(C₁-C₆)-alkylene, (C₁-C₆)-alkylene-O-(C₁-C₆)-alkyl, where one, more than one, or all hydrogen(s) in the alkyl, alkenyl, alkynyl and O-alkyl radicals may be replaced by fluorine;
NH₂, NH-(C₁-C₆)-alkyl, N((C₁-C₆)-alkyl)₂, NH-CO-(C₁-C₆)-alkyl,
or
R6 and R7 together with the C atoms carrying them a 5 to 7 membered, saturated, partially or completely unsaturated ring Cyc1, where 1 or 2 C atom(s) of the ring may also be replaced by N, O or S, and Cyc1 may optionally be substituted by (C₁-C₆)-alkyl, (C₂-C₅)-alkenyl, (C₂-C₅)-alkynyl, where in each case one CH₂ group may be replaced by O, or substituted by H, F, Cl, OH, CF₃, NO₂, CN, COO(C₁-C₄)-alkyl, CONH₂, CONH(C₁-C₄)-alkyl, CCF₃;
X CO, O, NH, a bond;
L (C₁-C₆)-alkylene, (C₂-C₅)-alkenylene, where in each case one or two CH₂ group(s) may be replaced by 0 or NH;
Y CO, NHCO, a bond.

5. A compound of the formula I as claimed in claims 1 to 4, in which the meanings are
R1 hydrogen;
R2 fluorine;
A O;
R3 CF₃, methyl, isopropyl;
R₄ hydrogen;
B (C₀-C₄)-alkylene, where one or more C atom(s) of the alkylene radical may be replaced independently of one another by -O-, -(C=O)-, -CHF- or -CF₂-;
X CO, O, a bond;
L (C₁-C₄) -alkylene, (C₂-C₄)-alkenylene, where in each case one or two CH₂ group(s) may be replaced by O or NH;
Y CO, NHCO, a bond.

6. A compound of the formula I as claimed in claims 1 to 5, in which the meanings are
R1 hydrogen;
R2 fluorine;
A O;
B -CH₂-;
R5 hydrogen, Cl, methyl, ethyl, OH, CF₃;
R6, R7 hydrogen;
X CO, O, a bond;
L (C₁-C₃) -alkylene, (C₂-C₃) -alkenylene, where in each case one CH₂ group may be replaced by O or NH;
Y CO, NHCO, a bond.

7. A compound of the formula I as claimed in claims 1 to 6, in which the substituents A and B occupy an adjacent position (ortho position) and R3 occupies an adjacent position (ortho position) to B.

8. A compound of the formula I as claimed in claims 1 to 7, in which the meanings are
R8, R9 independently of one another, hydrogen, SO₃H, sugar residue, (C₁-C₄)-alkyl, where the alkyl radical may be substituted independently of one another one or more times by (C₁-C₂)-alkyl, OH, (C₁-C₂)-alkylene-OH, OSO₃H, NH₂, CONH₂, SO₂NH₂, NH-SO₃H or adamantyl;
or
R8 and R9 together with the N atom carrying them form a 5 to 7 membered, saturated ring Cyc2, selected from the group of piperazine which may be N-substituted by (C₁-C₂) -alkyl, (C₁-C₂)-alkylene-OH or SO₃H,
piperidine, azepane, pyrrolidine or morpholine.

9. A compound selected from the group : 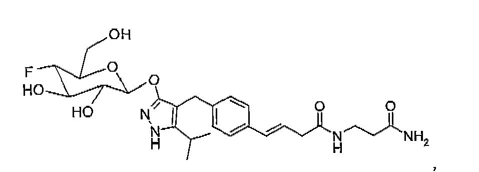 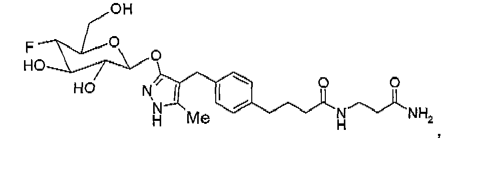 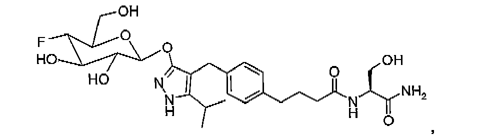 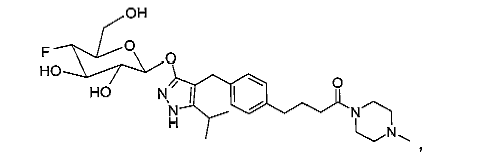 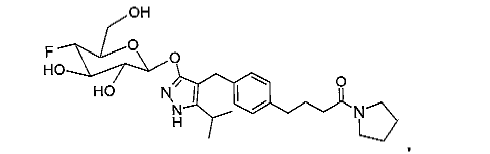 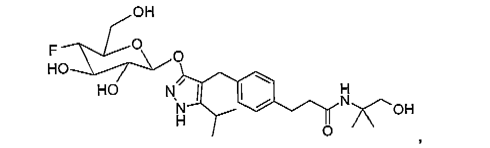 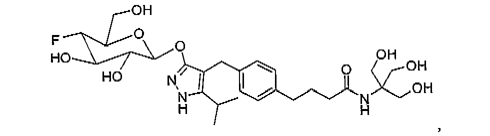 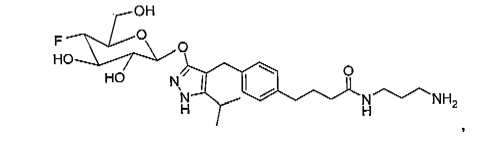 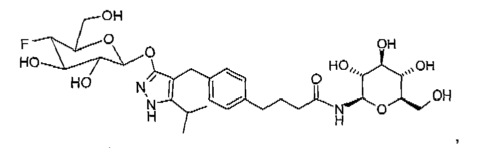 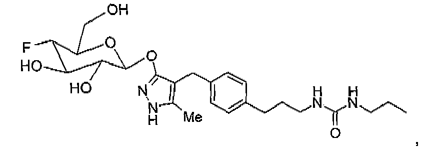 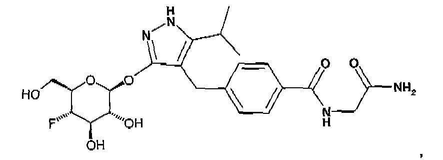 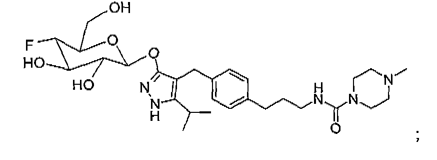 and the pharmaceutically acceptable salts thereof.

10. A medicament comprising one or more of the compounds as claimed in claims 1 to 9.

11. A medicament comprising one or more of the compounds as claimed in claims 1 to 9 and one or more blood glucose-lowering active ingredients.

12. The use of the compounds as claimed in claims 1 to 9 for producing a medicament.

13. The use of the compounds as claimed in claims 1 to 9 for producing a medicament for the treatment of type 1 and type 2 diabetes.

14. The use of the compounds as claimed in claims 1 to 9 for producing a medicament for lowering blood glucose.

15. The use of the compounds as claimed in claims 1 to 9 in combination with at least one further blood glucose-lowering active ingredient for producing a medicament for the treatment of type 1 and type 2 diabetes.

16. The use of the compounds as claimed in claims 1 to 9 in combination with at least one further blood glucose-lowering active ingredient for producing a medicament for lowering blood glucose.

17. A process for producing a medicament comprising one or more of the compounds as claimed in claims 1 to 9, which comprises mixing the active ingredient with a pharmaceutically suitable carrier, and converting this mixture into a form suitable for administration.

## Revendications

1. Composés de formule I 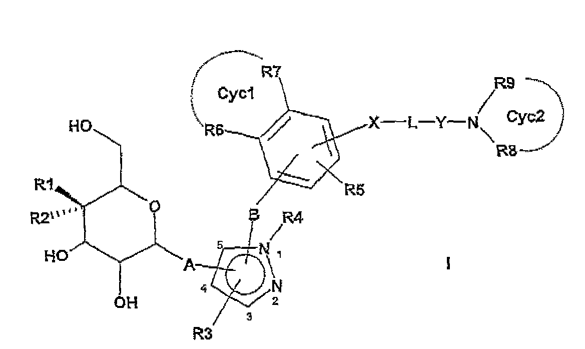 dans lesquelles les significations sont :
R1 et R2 indépendamment l'un de l'autre F ou H, où l'un des radicaux R1 ou R2 doit être F ;
A O, NH, CH₂, S ou une liaison ;
R3 hydrogène, F, Cl, Br, l, OH, CF₃, N0₂, CN, COOH, CO(C₁-C₆)-alkyle, COO(C₁-C₆)-alkyle, CONH₂, CONH(C₁-C₆)-alkyle, CON[(C₁-C₆)-alkyle]₂, (C₁-C₆)-alkyle, (C₃-C₆)-cycloalkyle, (C₂-C₆)-alcényle, (C₂-C₆)-alcynyle, O-(C₁-C₆)-alkyle, HO-(C₁-C₆)-alkylène, (C₁-C₆)-alkylène-O-(C₁-C₆)-alkyle, phényle, benzyle, (C₁-C₆)-alcoxycarboxyle, où un ou plusieurs ou l'ensemble des hydrogènes dans les radicaux alkyle, alcényle, alcynyle et O-alkyle peuvent être remplacés par fluor ;
SO₂-NH₂, SO₂NH(C₁-C₆)-alkyle, SO₂N[(C₁-C₆)-alkyle]₂, S-(C₁-C₆)-alkyle, S-(CH₂)₀-phényle, SO-(C₁-C₆)-alkyle, SO-(CH₂)ₒ-phényle, SO₂-(C₁-C₆)-alkyle, SO₂-(CH₂)ₒ-phényle, où o peut être 0 - 6 et le radical phényle peut être substitué jusqu'à deux fois par F, CI, Br, OH, CF₃, NO₂, CN, OCF₃, O-(C₁-C₆)-alkyle, (C₁-C₆)-alkyle, NH₂ ; NH₂, NH-(C₁-C₆)-alkyle, N((C₁-C₆)-alkyle)₂, NH-CO-(C₁-C₇)-alkyle, phényle, O-(CH₂)ₒ-phényle, où o peut être 0 - 6, où le cycle phényle peut être substitué de une à trois fois par F, CI, Br, l, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
R4 hydrogène, (C₁-C₆)-alkyle, (C₂-C₆)-alcényle, (C₃-C₆)-cycloalkyle ou phényle qui peut éventuellement être substitué par halogène ou (C₁-C₄)-alkyle ;
B (C₀-C₁₅)-alkylène, où un ou plusieurs atomes de C du radical alkylène peuvent être remplacés indépendamment les uns des autres par -O-, -(C=O)-, -CH=CH-, -C=C-, -S-, -CH(OH)-, -CHF-, -CF₂-, -(S=O)-, -(SO₂)-, -N-((C₁-C₆)-alkyle)-, -N((C₁-C₆)-alkylephényl)- ou -NH- ;
R5, R6, R7 indépendamment les uns des autres, hydrogène, F, Cl, Br, I, OH, CF₃, NO₂, CN, COOH, COO(C₁-C₆)-alkyle, CO(C₁-C₄)-alkyle, CONH₂, CONH(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₈)-alkyle, HO-(C₁-C₆)-alkylène, (C₁-C₆)-alkylène-O-(C₁-C₆)-alkyle, où un ou plusieurs ou l'ensemble des hydrogènes dans les radicaux alkyle, alcényle, alcynyle et O-alkyle peuvent être remplacés par fluor;
SO₂-NH₂, SO₂NH(C₁-C₆)-alkyle, SO₂N[(C₁-C₆)-alkyle]₂, S-(C₁-C₆)-alkyle, S-(CH₂)ₒ-phényle, SCF₃, SO-(C₁-C₆)-alkyle, SO-(CH₂)ₒ-phényle, SO₂-(C₁-C₆)-alkyle, SO₂-(CH₂)ₒ-phényle, où o peut être 0 - 6 et le cycle phényle peut être substitué jusqu'à deux fois par F, CI, Br, OH, CF₃, NO₂, CN, OCF₃, O-(C₁-C₆)-alkyle, (C₁-C₆)-alkyle, NH₂;
NH₂, NH-(C₁-C₆)-alkyle, N((C₁-C₆)-alkyle)₂, NH-CO-(C₁-C₆)-alkyle, phényle, O-(CH₂)ₒ-phényle, où o peut être 0 - 6, où le cycle phényle peut être substitué de une à trois fois par F, CI, Br, I, OH, CF₃, NO₂, CN, OCF₃, O-(C₁-C₈)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
ou
R6 et R7 conjointement avec les atomes de C les portant, un cycle Cyc1 de 5 à 7 chaînons, saturé, partiellement ou totalement insaturé, où 1 ou 2 atomes de C du cycle peuvent également être remplacés par N, O ou S, et Cyc1 peut éventuellement être substitué par (C₁-C₆)-alkyle, (C₂-C₅)-alcényle, (C₂-C₅)-alcynyle, où dans chaque cas un groupement CH₂ peut être remplacé par O ou substitué par H, F, CI, OH, CF₃, NO₂, CN, COO(C₁-C₄)-alkyle, CONH₂, CONH(C₁-C₄)-alkyle, OCF₃ ;
X CO, O, NH, S, SO, SO₂ ou une liaison ;
L (C₁-C₆)-alkylène, (C₂-C₅)-alcénylène, (C₂-C₅)-alcynylène, où dans chaque cas un ou deux groupements CH₂ peuvent être remplacés par O ou NH;
Y CO, NHCO, SO, SO₂ ou une liaison ;
R8, R9 indépendamment l'un de l'autre, hydrogène, SO₃H, résidu de sucre, (C₁-C₆)-alkyle, où un ou plusieurs groupements CH₂ du radical alkyle peuvent être substitués indépendamment les uns des autres par (C₁-C₆)-alkyle, OH, (C₁-C₆)-alkylène-OH, (C₂-C₆)-alcénylène-OH, O-résidu de sucre, OSO₃H, NH₂, NH-(C₁-C₆)-alkyle, N[(C₁-C₆)-alkyle]₂, NH-CO-(C₁-C₆)-alkyle, NH-résidu de sucre, NH-SO₃H, (C₁-C₆)-alkylène-NH₂, (C₂-C₆)-alcénylène-NH₂, (C₀-C₆)-alkylène-COOH, (C₀-C₆)-alkylène-CONH₂, (C₀-C₆)-alkylène-CONH-(C₁-C₆)-alkyle, (C₀-C₆)-alkylène-SONH₂, (C₀-C₆)-alkylène-SONH-(C₁-C₆)-alkyle, (C₀-C₆)-alkylène-SO₂NH₂, (C₀-C₆)-alkylène-SO₂NH-(C₁-C₆)-alkyle, adamantyle ; ou
R8 et R9 conjointement avec l'atome de N les portant, forment un cycle Cyc2 de 5 à 7 chaînons, saturé, où un ou plusieurs groupements CH₂ du cycle peuvent également être remplacés par O, S, NH, NSO₃H, un N-résidu de sucre, N-(C₁-C₆)-alkyle, où un ou plusieurs groupements CH₂ du radical alkyle peuvent être substitués indépendamment les uns des autres par (C₁-C₆)-alkyle, OH, (C₁-C₆)-alkylène-OH, (C₂-C₆)-alcénylène-OH, NH₂, NH-(C₁-C₆)-alkyle, N[(C₁-C₆)-alkyle]₂, NH-CO-(C₁-C₆)-alkyle, NH-résidu de sucre, (C₁-C₆)-alkylène-NH_{2'} (C₂-C₆)-alcénylène-NH₂, (C₀-C₆)-alkylène-COOH, (C₀-C₆)-alkylène-CONH₂, (C₀-C₆)-alkylène-CONH-(C₁-C₆)-alkyle, (C₀-C₆)-alkylène-SONH₂, (C₀-C₆)-alkylène-SONH-(C₁-C₆)-alkyle, (C₀-C₆)-alkylène-SO₂NH₂, (C₀-C₆)-alkylène-SO₂NH-(C₁-C₆)-alkyle ;
et les sels pharmaceutiquement acceptables de celui-ci.

2. Composés de formule 1 selon la revendication 1, **caractérisé en ce que** les significations sont
A O, NH, une liaison ;
R3 hydrogène, F, Cl, Br, I, OH, CF₃, NO₂, CN, COOH, CO-(C₁-C₆)-alkyle, COO-(C₁-C₆)-alkyle, CONH₂, CONH-(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₆)-alkyle, HO-(C₁-C₆)-alkylène, (C₁-C₆)-alkylène-O-(C₁-C₆)-alkyle, phényle, benzyle, où un ou plusieurs ou l'ensemble des hydrogènes dans les radicaux alkyle peuvent être remplacés par fluor, SO-(C₁-C₆)-alkyle; ou
R4 hydrogène, (C₁-C₆)-alkyle, (C₂-C₆)-alcényle, (C₃-C₆)-cycloalkyle ;
B (C₀-C₆)-alkylène, où un ou plusieurs atomes de C du radical alkylène peuvent être remplacés indépendamment les uns des autres par -O-, -(C=O)-, -CH=CH-, -C≡C-, -S-, -CH(OH)-, -CHF-, -CF₂-, -(S=O)-, -(SO₂)-, -N-((C₁-C₆)-alkylène)-, -N((C₁-C₆)-alkylène-phénylène)- ou -NH-.

3. Composés de formule I selon la revendication 1 ou 2, **caractérisé en ce que** les résidus de sucre sont liés en bêta (β) et la stéréochimie dans les positions 2, 3 et 5 du résidu de sucre a la configuration de D-gluco.

4. Composés de formule I selon les revendications 1 à 3, **caractérisé en ce que** les significations sont
R1 hydrogène et
R2 fluor;
ou
R1 fluor et
R2 hydrogène ;
A O,NH;
R3 hydrogène, F, Cl, Br, I, OH, CF₃, (C₁-C₆)-alkyle, (C₃-C₆)-cycloalkyle, (C₂-C₆)-alcényle, O-(C₁-C₆)-alkyle, où un ou plusieurs ou l'ensemble des hydrogènes dans les radicaux alkyle peuvent être remplacés par fluor ;
R4 hydrogène, (C₁-C₆)-alkyle, (C₃-C₆)-cycloalkyle ;
B (C₀-C₄)-alkylène, où un ou plusieurs atomes de C du radical alkylène peuvent être remplacés indépendamment les uns des autres par -O-, -(C=O)-, -CH=CH-, -CH(OH)-, -CHF-, -CF₂- ou -NH- ;
R5, R6, R7 indépendamment les uns des autres, hydrogène, F, CI, Br, l, OH, CF₃, N0₂, CN, COOH, COO(C₁-C₆)-alkyle, CO(C₁-C₄)-alkyle, CONH₂, CONH-(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₈)-alkyle, HO-(C₁-C₆)-alkylène, (C₁-C₆)-alkylène-O-(C₁-C₆)-alkyle, où un ou plusieurs ou l'ensemble des hydrogènes dans les radicaux alkyle, alcényle, alcynyle et O-alkyle peuvent être remplacés par fluor ;
NH₂, NH-(C₁-C₆)-alkyle, N((C₁-C₆)-alkyle)₂, NH-CO-(C₁-C₆)-alkyle;
ou
R6 et R7 conjointement avec les atomes de C les portant, un cycle Cyc1 de 5 à 7 chaînons, saturé, partiellement ou totalement insaturé, où 1 ou 2 atomes de C du cycle peuvent également être remplacés par N, O ou S, et Cyc1 peut éventuellement être substitué par (C₁-C₆)-alkyle, (C₂-C₅)-alcényle, (C₂-C₅)-alcynyle, où dans chaque cas un groupement CH₂ peut être remplacé par O ou substitué par H, F, CI, OH, CF₃, NO₂, CN, COO(C₁-C₄)-alkyle, CONH₂, CONH(C₁-C₄)-alkyle, OCF₃;
X CO, O, NH, une liaison ;
L (C₁-C₆)-alkylène, (C₂-C₅)-alcénylène, où dans chaque cas un ou deux groupements CH₂ peuvent être remplacés par O ou NH ;
Y CO, NHCO, une liaison.

5. Composés de formule 1 selon les revendications 1 à 4, **caractérisé en ce que** les significations sont
R1 hydrogène ;
R2 fluor;
A O;
R3 CF₃, méthyle, isopropyle ;
R4 hydrogène ;
B (C₀-C₄)-alkylène, où un ou plusieurs atomes de C du radical alkylène peuvent être remplacés indépendamment les uns des autres par -O-, -(C=O)-, -CHF- ou -CF₂- ;
X CO, O, une liaison ;
L (C₁-C₄)-alkylène, (C₂-C₄)-alcénylène, où dans chaque cas un ou deux groupements CH₂ peuvent être remplacés par O ou NH;
Y CO, NHCO, une liaison.

6. Composés de formule 1 selon les revendications 1 à 5, **caractérisé en ce que** les significations sont
R1 hydrogène ;
R2 fluor;
A O;
B -CH₂-;
R5 hydrogène, CI, méthyle, éthyle, OH, CF₃ ;
R6, R7 hydrogène ;
X CO, O, une liaison ;
L (C₁-C₃)-alkylène, (C₂-C₃)-alcénylène, où dans chaque cas un groupement CH₂ peut être remplacé par O ou NH ;
Y CO, NHCO, une liaison.

7. Composés de formule 1 selon les revendications 1 à 6, **caractérisé en ce que** les substituants A et B occupent une position adjacente (position ortho) et R3 occupe une position adjacente (position ortho) à B.

8. Composés de formule I selon les revendications 1 à 7, **caractérisé en ce que** les significations sont
R8, R9 indépendamment l'un de l'autre, hydrogène, SO₃H, résidu de sucre, (C₁-C₄)-alkyle, où le radical alkyle peut être substitué indépendamment l'un de l'autre une ou plusieurs fois par (C₁-C₂)-alkyle, OH, (C₁-C₂)-alkylène-OH, OSO₃H, NH₂, CONH₂, SO₂NH₂, NH-SO₃H ou adamantyle ;
ou
R8 et R9 conjointement avec l'atome de N les portant, forment un cycle Cyc2 de 5 à 7 chaînons, saturé, choisi dans le groupe de pipérazine qui peut être N-substituée par (C₁-C₂)-alkyle, (C₁-C₂)-alkylène-OH ou SO₃H, pipéridine, azépane, pyrrolidine ou morpholine.

9. Composé choisi parmi le group: 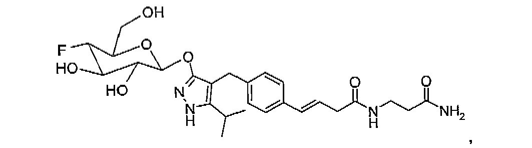 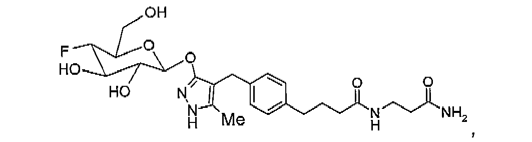 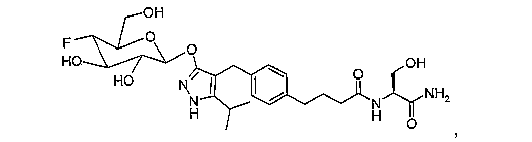 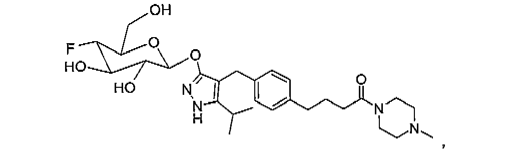 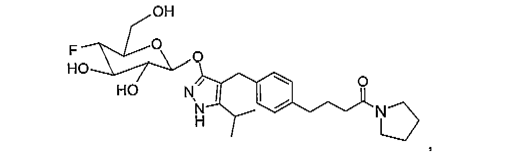 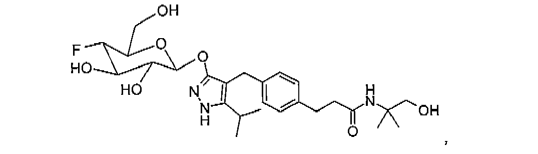 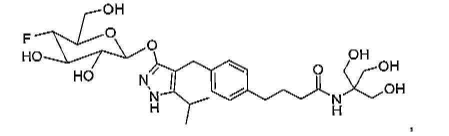 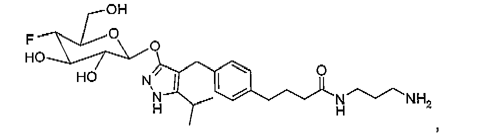 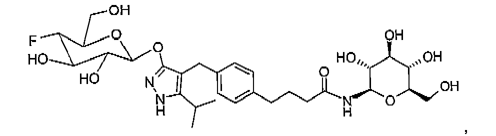 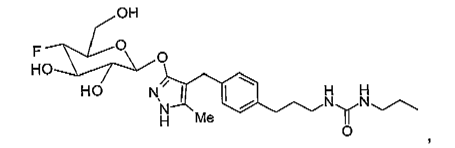 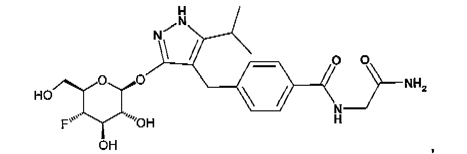 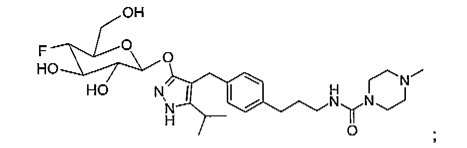 et les sels pharmaceutiquement acceptables de celui-ci.

10. Médicament comprenant un ou plusieurs des composés selon les revendications 1 à 9.

11. Médicament comprenant un ou plusieurs des composés selon les revendications 1 à 9 et un ou plusieurs principes actifs abaissant le taux de glucose sanguin.

12. Utilisation des composés selon les revendications 1 à 9, pour la production d'un médicament.

13. Utilisation des composés selon les revendications 1 à 9, pour la production d'un médicament destiné au traitement du diabète de type 1 et de type 2.

14. Utilisation des composés selon les revendications 1 à 9, pour la production d'un médicament destiné à baisser le taux de glucose sanguin.

15. Utilisation des composés selon les revendications 1 à 9, en combinaison avec au moins un autre principe actif abaissant le taux de glucose sanguin, pour la production d'un médicament destiné au traitement du diabète de type 1 et de type 2.

16. Utilisation des composés selon les revendications 1 à 9, en combinaison avec au moins un autre principe actif abaissant le taux de glucose sanguin, pour la production d'un médicament destiné à baisser le taux de glucose sanguin.

17. Procédé de production d'un médicament comprenant un ou plusieurs des composés selon les revendications 1 à 9, **caractérisé en ce que** le principe actif est mélangé avec un support pharmaceutiquement convenable, et ce mélange est transformé en une forme convenable pour l'administration.
